# EUROPEAN PATENT SPECIFICATION

(11) **EP 4 469 499 B1**
(45) Date of publication and mention of the grant of the patent: **09.09.2026**
(21) Application number: 23709912.2
(22) Date of filing: 25.01.2023
(51) Int. Cl.: C08G 18/63, C08G 18/48, C08G 18/76, C07C 4/22, C08J 11/12, C08L 51/00, C08F 261/06, C08F 283/06

(54) **NON AQUEOUS POLYMERIC DISPERSION OF VINYL MONOMERS PRODUCED FROM POLYSTYRENE PYROLYSIS OIL**
NICHTWÄSSRIGE POLYMERE DISPERSION VON VINYLMONOMEREN, HERGESTELLT AUS POLYSTYROL-PYROLYSEÖL
DISPERSION POLYMÉRIQUE NON AQUEUSE DE MONOMÈRES VINYLIQUES PRODUITS À PARTIR D'HUILE DE PYROLYSE DE POLYSTYRÈNE

(30) Priority: 26.01.2022 EP 22382055
(43) Date of publication of application: 04.12.2024
(73) Proprietor: Repsol, S.A., 28045 Madrid (ES)
(72) Inventor: PÉREZ VALENCIA, Juan Pedro, 28935 MÓSTOLES (ES); DOMÍNGUEZ RAMOS, Enrique, 28935 MÓSTOLES (ES)
(74) Representative: ZBM Patents - Zea, Barlocci & Markvardsen
(86) International application number: PCT/EP2023/051740
(87) International publication number: WO 2023/144170

(56) References cited:
- EP-A1- 1 624 006
- WO-A1-2021/053074
- WO-A1-2021/241377
- US-A1- 2013 281 627

## Description

This invention relates to a polymer polyol comprising a dispersed phase formed by polymeric particles which are the polymerization product of at least polystyrene pyrolysis oil, and acrylonitrile; and to compositions comprising or made of the polymer polyol (particularly polyurethane (foams)). The invention also relates to a process for preparing polymer polyols using polystyrene pyrolysis oil; as well as processes for the preparation of the compositions comprising or made from the polymer polyol.

### Background Art

Polymer polyols (also known filled polyols) are high volume commercial products whose main use is the production of polyurethane foams. Polymer polyols contain dispersions of particles of a polymer formed from the polymerization of selected monomeric compounds (monomers), such as acrylonitrile, styrene, and vinyl chloride in liquid polyol. Commercially, the most important products are based on acrylonitrile and styrene (SAN). The presence of the polymeric particles in the polyol imparts various desirable properties to polyurethane foams, such as reinforcing fillers and cell openers.

Polymer polyols are commonly prepared by *de novo* synthetic processes such as dispersion polymerization processes, which first involves the production of radicals resulting from the thermal decomposition of a free-radical (polymerization) initiator, which in turn reacts with the vinylic monomer(s) to form growing oligo-radicals. Depending on the solubility of those oligo-radicals in the medium, each oligo-radical can collapse into a condensed state when a certain threshold molecular weight is reached, giving rise to primary particles which attract either other primary particles or already existing larger ones. Typically, azo compounds and peroxides are used as catalytic polymerising initiators and the reaction can take place at temperatures from 80-130°C, wherein monomer(s) are added to polyol at such a rate that its concentration remains low throughout the process. Further, chain transfer agents are generally used to control molecular weight and grafting of the polyol.

In order to improve the stability of the polymer polyol dispersions, stabilizers or dispersants are generally used. The type of stabilizer and/or its concentration, may determine the particle size and particle size distribution of the dispersion which, in turns affects the polymer polyol properties such as viscosity. The most successful type of dispersant devised for use in dispersion polymerization has been based on a block or graft copolymer which consists of two essential polymeric components, one soluble and one insoluble (or anchor group) in the continuous phase. The insoluble component associates with the disperse phase polymer becoming physically absorbed into the polymeric particle or reacting chemically with the disperse phase after absorption.

Alternatively, the dispersant may be either preformed or formed in situ. In any of these cases, a precursor is usually employed. This precursor is also known as "macromonomer" or "macromer", which are polyether polyols (identical or different to the liquid polyol) with terminal double bonds, able to copolymerize with the monomer(s) and to form graft species during the radical copolymerization. The polyol part typically contains long chains that are highly soluble in the continuous phase of the polymer polyol. In fact, the resulting block copolymer after reacting the macromer with vinylic monomers is a non-aqueous dispersant which introduces polyol-soluble moieties onto the copolymer particles leading to improved particle stability.

Thus, polymer polyol processes can be divided into "in situ" formation simultaneously to polymer polyol synthesis process and preformed stabilizer synthesis process depending on dispersant synthesis. On one hand, in an "in situ" formation simultaneously to polymer polyol synthesis process, the macromer is added to the organic liquid serving as the polymerization medium (liquid polyol). The monomer system being polymerized reacts with the macromer during polymerization to form, in situ, a graft or addition copolymer dispersant. Thus, this process involves the simultaneous dispersion polymerization of monomers to produce polymeric particles and block copolymer dispersant formation by grafting reaction of a macromer and monomers. On the other hand, in the preformed stabilizer synthesis process, the graft copolymer dispersant synthesis takes place apart from the main polymerization process. Reaction procedure is similar to polymer polyol synthesis (it uses the same or similar reaction scheme, initiator, chain transfer agent, monomers...) but employing different concentrations and similar or different reaction conditions leading to a concentrated preformed stabilizer product which is added to polymer polyol reaction process. Besides, the above mentioned polymer polyol processes can be performed in batch, semibatch or continuous way. The continuous processes are favoured when the dispersant is pre-formed as a step prior to the reaction leading to the obtention of the polymeric polyol.

Polymer polyols having both a relatively high solid polymer content and a sufficiently low viscosity for ease of handling are favourable for the preparation of polyurethane foams with appropriate properties. High level of dispersed polymeric particles (a concentrated polymer polyol) provides enhanced reinforcement and cell opening as mentioned above. In addition, the production of high level solids polymer polyols increases productivity since it is possible to get products containing smaller amounts just by diluting the concentrated product.

However, problems have been typically found in the manufacture of these polymer polyols having relatively high solid polymer content and an appropriate viscosity value. One of the problems is that the dispersed polymer particles tend to agglomerate and then settle out of the continuous polyol phase invalidating its use. Another problem is the exponential increase in viscosity up to a limit that makes it impossible to be processed at acceptable rate or accuracy using the usual pumping equipment for the foamers. Another further problem is the control of the particle size of the dispersed particles which is critical since the presence of large particles may affect foam mechanical properties meanwhile the presence of small particles could also be detrimental for its viscosity.

Finally, one of the most serious problems for the preparation of concentrated polymer polyol is its high susceptibility to the presence of impurities or component foreign to the process. Commonly. impurities may be present in reactants, or they may be formed as by-products during the production of the polymer polyol. It is known in the state of the art that impurities (particularly those impurities accompanying the styrene monomer) can affect seriously the quality, and particularly, the filterability of polymer polyols. Typical impurities in styrene monomer include, for example, compounds which contain an aromatic ring such as benzene, ethyl benzene, benzaldehyde, phenylacetylene, xylenes and polystyrene. These impurities, among others, are typical by-products that occur during the process of producing styrene monomer.

Furthermore, the presence of these impurities or component foreign to the process in the resulting polymer polyol are also critical for the subsequent preparation of foams, such as polyurethane. As it is known in the state of the art, foam preparation processes are very sensitive to the reaction conditions and to the presence of impurities. In fact, their presence can affect drastically to the process and the quality of the foam thus obtained, by causing from the collapse of the foam to malformations of the cellular structure.

In order to solve the above mentioned problems, the reduction of impurities and/or by-products, particularly those which contain at least one aromatic ring (below 1000 ppm) has been found to be crucial for the preparation of concentrated polymer polyol having good stability, appropriate processability properties and polyurethane foaming results. In fact, it has been correlated that when the quantity of impurities presents in the styrene monomer increase, the quality of the polymer polyol is negatively impacted (cf. WO2014/035782). However, the use of high purity reagents for the process and the reduction of some impurities and/or by-products in the resulting polymer polyol makes its industrial production material, energy, and capital intensive.

Furthermore, for the past fifty years, styrene-based polymers and their derivatives have played a crucial role in upgrading the human society. It has imparted impulse in the development of numerous sectors like packaging, electronics, automobiles, medical, construction, among others. Due to the swift surge in the global population, the market need for these products has hugely risen. The regular increase in the requirement of these type of styrene-based compounds and plasticware products formed from them, led to a waste accretion every year. These data signify that the quantity of plastic scrap that has accumulated in landfills is too large. As a fact, natural degradation of them may take billions of years. Consequently, the regular accretion of plastic in the landfill poses a severe environmental hazard. And, therefore, the manufacturing of polymer polyol by a de novo process, even less using highly pure starting material is not environmentally recommended. To curtail this accumulation, recycling techniques has been developed. However, nowadays, recycling the plastic turns out to be a cumbersome and costly process since it requires complexes methods with a complicated labour to obtain again "pure" products.

Therefore, from what it is known in the state of the art, there is still the need of providing an effective and environmentally friendly process for the preparation of circular polymer polyol having a good stability and processability properties, particularly appropriate for the manufacturing of polyurethane foams.

### Summary of Invention

Inventors have surprisingly found that a pyrolysis oil of polystyrene enriched in styrene monomer is appropriate for the preparation of polymer polyol. Particularly, the inventors have found that the partial or total substitution of the pure styrene monomer for a pyrolysis oil of polystyrene enriched in styrene monomer does not affect both the polymerization process (for instance polymerization conversion) and the properties of the polymer polyol thus obtained.

In fact, the use of polystyrene pyrolysis oil allows obtaining the polymer polyol of the present invention that comprises a dispersed phase formed by polymeric particles comprising monomeric units of styrene, acrylonitrile, and alpha-methyl styrene; and a continuous phase comprising a polyether polyol. Furthermore, as it is demonstrated in the experimental section, the polymer polyol of the present invention has a stability, resistance and processability properties comparable to those SAN polymer polyols disclosed in the state of the art (cf. experimental data section 1.2).

As it is disclosed in the state of the art, the oils obtained from the pyrolysis of polystyrene are a complex mixture formed by a lot of compounds that can be potentially incompatibles with a catalytic polymerization reaction (such as those used for the preparation of polymer polyol). The pyrolysis oil contains components considered impurities, by-products, additives, and/or radical initiator poisoners among others, making the pyrolysis oil theoretically incompatible with a styrene polymerization, as it happens in polystyrene polymerization processes, wherein the polystyrene can be reproduced from liquid fractions of pyrolysis oil of polystyrene but with inferior properties compared to a polystyrene prepared from neat styrene (pure styrene)(see. Recycling of Polymers. Methods, Characterization and Applications. Ed. Raju Francis. 2017 Wiley-VCH. p. 81).

Nevertheless, contrary to the knowledge of the state of the art, as it is demonstrated in the experimental section, the use of a pyrolysis oil of polystyrene containing the determined quantity of styrene mentioned in the present application allows obtaining a polymer polyol with an appropriate particle size, viscosity, and solid content that in turn allows preparing polyurethane foams from them without compromising neither the synthesis process nor their final properties.

Particularly, it was surprisingly observed that the conversion of the polymerization process of the polymer polyol is performed by using pyrolysis oil of polystyrene but without the need of increasing the amount of radical initiator, and even using a lower amount of change transfer agent. Furthermore, the viscosity value of the polymer polyol obtained by the use of the pyrolysis oil without further purification (pyrolysis oil 1) is lower than those obtained with the comparative process and therefore, the polymer polyol of the invention (cf. Ex.2) has better processability properties for the preparation of polyurethane foams.

Then, the use of pyrolysis oil of polystyrene is advantageous because allows preparing efficiently circular polymer polyols using an oil which does not require to perform costly and tedious fractionating/purifying steps. Thus, the polymer polyol and the process for its preparation can be considered environmentally friendly, reducing the synthesis de novo of styrene monomer and/or offering means for polystyrene waste treatment different from incineration and/or dump.

Furthermore, the pyrolysis oil can be used in already existing equipment without being modified, because it is compatible with the commonly used equipment for the preparation of polymer polyol either in batch, semibatch or in continuous processes without dirtying or ruining the equipment. It means that there is no need of adapting the existing equipment, it is only needed to substitute all or part of the amount of pure styrene monomer for the pyrolysis oil of polystyrene.

Then, the first aspect of the invention relates to a polymer polyol comprising: (a) a dispersed phase formed by polymeric particles (a1) which are the polymerization product of a polystyrene pyrolysis oil, and acrylonitrile; or alternatively, of a polystyrene pyrolysis oil, acrylonitrile, and styrene monomer; and (b) a continuous phase comprising:
(b1) a polyol; and (b2) optionally, one or more of the compounds selected from the group consisting of macromer; dispersant; 2,4-diphenyl-1-butene and isomers thereof; 2,4,6-triphenyl-1-hexene and isomers thereof; 2-phenylnaphthalene; and poly-aromatic compounds; wherein the polystyrene pyrolysis oil comprises from 50% to 98% by weight of styrene monomer.

In particular, the invention relates to a polymer polyol comprising: (a) a dispersed phase formed by polymeric particles (a1) which are the polymerization product of a polystyrene pyrolysis oil, and acrylonitrile; or alternatively, of a polystyrene pyrolysis oil, acrylonitrile, and styrene monomer; and (b) a continuous phase comprising: (b1) a polyol; and (b2) optionally, one or more of the compounds selected from the group consisting of macromer; dispersant; 2,4-diphenyl-1-butene and isomers thereof; 2,4,6-triphenyl-1-hexene and isomers thereof; 2-phenylnaphthalene; and poly-aromatic compounds; wherein the polystyrene pyrolysis oil comprises from 50% to 98% by weight of styrene monomer, and the polymeric particles comprise: monomeric units of styrene, acrylonitrile, and alpha-methyl styrene; and one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and isomers thereof.

The second aspect of the invention is a process for the preparation of a polymer polyol comprising: (a) a dispersed phase formed by polymeric particles (a1); and (b) a continuous phase comprising a polyol (b1); wherein the polymeric particles (a1) comprise: monomeric units of styrene, acrylonitrile, and alpha-methyl styrene; and one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and isomers thereof; and wherein: the process comprises: (i) providing polymeric particles dispersed in a continuous polyol phase by polymerization of a polystyrene pyrolysis oil, and acrylonitrile; or alternatively by polymerization of a polystyrene pyrolysis oil, acrylonitrile, and styrene monomer; and one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene and isomers thereof; and 2,4,6-triphenyl-1-hexene and isomers thereof; in a continuous polyol phase in the presence of at least one radical initiator; wherein the polystyrene pyrolysis oil comprises from 50% to 98% by weight of styrene monomer. Particularly, the process of the second aspect of the invention allows preparing the polymer polyol of the first aspect of the invention. Thus, a polymer polyol of the first aspect of the invention obtained by the process of the second aspect of the invention is also part of the present application.

The third aspect of the invention relates to compositions comprising the polymer polyol as defined in the first aspect of the invention and one or more excipients or carriers; or alternatively, a composition made at least from the polymer polyol as defined in the first aspect of the invention.

And the fourth aspect of the invention is the use of polymer polyol of the first aspect of the invention for the preparation of polyurethane (particularly polyurethane foams) and a process for their preparation.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the present invention, any ranges given include both the lower and the upper end-points of the range. Ranges and values given, such as temperatures, times, and the like, should be considered approximate, unless specifically stated.

The terms "percentage (%) by weight" or "percentage (%) w/w" or "%wt" have the same meaning and are used interchangeable. This term refers to the percentage of a component in relation to the total weight.

The term "about" or "around" as used herein refers to a range of values ± 10% of a specified value. For example, the expression "about 35" or "around 35" includes ± 10% of 35, i.e. from 31.5 to 38.5.

As it is mentioned above, the first aspect of the invention relates to polymer polyol comprising:
(a) a dispersed phase formed by polymeric particles (a1) which are the polymerization product of a polystyrene pyrolysis oil, and acrylonitrile; or alternatively, of a polystyrene pyrolysis oil, acrylonitrile, and styrene monomer;
   and
(b) a continuous phase comprising:
   (b1) a polyol; and
   (b2) optionally, one or more of the compounds selected from the group consisting of macromer; dispersant; 2,4-diphenyl-1-butene and isomers thereof; 2,4,6-triphenyl-1-hexene and isomers thereof; 2-phenylnaphthalene; and poly-aromatic compounds; wherein the polystyrene pyrolysis oil comprises from 50% to 98% by weight of styrene monomer.

In particular, the invention relates to a polymer polyol comprising: (a) a dispersed phase formed by polymeric particles (a1) which are the polymerization product of a polystyrene pyrolysis oil, and acrylonitrile; or alternatively, of a polystyrene pyrolysis oil, acrylonitrile, and styrene monomer; and (b) a continuous phase comprising: (b1) a polyol; and (b2) optionally, one or more of the compounds selected from the group consisting of macromer; dispersant; 2,4-diphenyl-1-butene and isomers thereof; 2,4,6-triphenyl-1-hexene and isomers thereof; 2-phenylnaphthalene; and poly-aromatic compounds; wherein the polystyrene pyrolysis oil comprises from 50% to 98% by weight of styrene monomer, and the polymeric particles comprise: monomeric units of styrene, acrylonitrile, and alpha-methyl styrene; and one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and isomers thereof.

In an embodiment, the polymer polyol optionally comprises (b2) one or more of the compounds selected from the group consisting of macromer; dispersant; 2,4-diphenyl-1-butene and isomers thereof; 2,4,6-triphenyl-1-hexene and isomers thereof; 2-phenylnaphthalene; and poly-aromatic compounds; wherein the poly-aromatic compounds are selected from the group consisting of diphenyl, bibenzyl, diphenyl propane, naphthalene, and mixtures thereof.

In an embodiment, the polymer polyol comprises (b2) one or more of the compounds selected from the group consisting of macromer; dispersant; 2,4-diphenyl-1-butene and isomers thereof; 2,4,6-triphenyl-1-hexene and isomers thereof; 2-phenylnaphthalene; and poly-aromatic compounds; particularly the poly-aromatic compounds are selected from the group consisting of diphenyl, bibenzyl, diphenyl propanes, naphthalene and mixtures thereof.

In an embodiment, the polymer polyol is selected from the group consisting of polymer polyether polyol, polymer polyester polyol, polymer polycarbonate polyol, polymer polyether carbonate polyol and a mixture thereof. The appropriate starting polymer, reagents, and their amounts as well as the process and reaction conditions for its preparation can readily be determined by those skilled in the art according to the field, the type and amount of polymer polyol being prepared; and the composition finally produced containing it or made from it.

In an embodiment, the polyol (b1) is a "polyether polyol" also usually referred to as "polyalkylene polyether polyol" or "polyoxyalkylene polyols". In general, polyether polyol is the product of polymerization of one or more alkylene oxides in the presence of one or more initiator or starter compound in the presence of a (basic) catalyst. Suitable starter or initiators for the polyoxyalkylene polyols which can be used are the alkylene oxide adducts of a variety of suitable initiator molecules. Non-limiting examples include dihydric initiators such as ethylene glycol, diethylene glycol, triethylene glycol, propylene glycol, dipropylene glycol, tripropylene glycol, neopentyl glycol, 1,3-propanediol, 1,4-butanediol, 1,6-hexanediol, 1,4-cyclo-hexanediol, 1,4-cyclohexane-dimethanol, hydroquinone, hydroquinone bis(2-hydroxyethyl)ether, the various bisphenols such as bisphenol A and bisphenol F and their bis(hydroxyalkyl)ether derivatives, aniline, the various N-N-bis(hydroxyalkyl)anilines, primary alkyl amines and the various N-N-bis(hydroxyalkyl)amines; trihydric initiators such as glycerin, trimethylolpropane, trimethylolethane, the various alkanolamines such as ethanolamine, diethanolamine, triethanolamine, propanolamine, dipropanolamine, and tripropanolamine; tetrahydric initiators such as pentaerythritol, ethylene diamine, N,N,N',N'-tetrakis[2-hydroxyalkyl]ethylenediamines, toluene diamine and N,N,N',N'-tetrakis[hydroxy-alkyl]toluene diamines; pentahydric initiators such as the various alkyl glucosides, particularly α-methyl glucoside; hexahydric initiators such as sorbitol, mannitol, hydroxyethyl glucoside, and hydroxypropyl glucoside; octahydric initiators such as sucrose; and higher functionality initiators such as various starch and partially hydrolyzed starch-based products, and methylol group-containing resins and novolak resins such as those prepared from the reaction of as aldehyde, preferably formaldehyde, with a phenol, cresol, or other aromatic hydroxyl-containing compound.

In an embodiment, the polyether polyol is the product of polymerization of alkylene oxides having from 2 to 4 carbon atoms (e.g. ethylene oxide, propylene oxide, butylene oxide and mixtures thereof) in the presence of a starting compound having at least one active hydrogen atoms. Suitable starting compounds for the obtention of polyether polyols include, for example, polyhydroxyl compounds such as, ethylene glycol, propylene glycol, diethylene glycol, dipropylene glycol, tripropylene glycol, trimethylolpropane, glycerol, sorbitol, glucose, and sucrose; water; ammonia; amino alcohols such as ethanolamine, diethanolamine, triethanolamine; and primary and/or secondary amine or polyamines such as ethylenediamine, aniline and toluene diamine. By alkoxylation of the starter, a suitable polyether polyol can be formed. The alkoxylation reaction may be catalysed using any conventional catalyst including, for example, potassium hydroxide or a double metal cyanide (DMC) catalyst. Examples of suitable polyether polyols include polyoxyethylene glycols, triols, tetrols and higher functionality polyols; polyoxypropylene glycols, triols, tetrols and higher functionality polyols; and mixtures thereof. When ethylene oxide and propylene oxide mixtures are used to produce the polyether polyol, the ethylene oxide and propylene oxide may be added simultaneously or sequentially so that the polyether polyol has internal blocks, terminal blocks, or a random distribution of oxyethylene groups and/or oxypropylene groups.

Other polyether polyols suitable for use as the polyol of the present invention include: alkylene oxide adducts of 1,3-dihydroxypropane, 1,3-dihydroxybutane, 1,4-dihydroxybutane, 1,4-, 1,5-, 1,6-dihydroxyhexane, 1,2-, 1,3-, 1,4-, 1,6-, 1,8-dihydroxyoctant, 1,10-dihydroxydecane, glycerol, 1,2,4-trihydroxybutane, 1,2,6-trihydroxyhexane, 1,1,1-trimethyl-olethane, 1,1,1-trimethylol propane, pentaerythritol, caprolactone, polycaprolactone, xylitol, arabitol, sorbitol, mannitol, and the like. Other polyether polyols which can be used as a polyol include the alkylene oxide adducts of non-reducing sugars, wherein the alkylene oxides have from 2 to 4 carbon atoms. Non-reducing sugars and sugar derivatives include sucrose, alkyl glycosides such as ethylene glycol glycoside, propylene glycol glucoside, glycerol glucoside, and 1,2,6-hexanetriol glucoside, as well as alkylene oxide adducts of the alkyl glycosides. Other polyether suitable polyols include the polyphenols and particularly alkylene oxides adducts thereof in which the alkylene oxides have from 2 to 4 carbon atoms. Among the suitable polyphenols are bisphenol A, bisphenol F, condensation products of phenol and formaldehyde, the novolac resins, condensation products of various phenolic compounds and acrolein, including the 1,1,3-tris(hydroxyl-phenyl)propanes, condensation products of various phenolic compounds and glyoxal, glutaraldehyde, and other dialdehydes, including the 1,1,2,2-tetrakis(hydroxyphenol)ethanes.

In an embodiment, the polyol is a polyether polyol built up of propylene oxide and ethylene oxide units and particularly having a molecular weight from 2.000 to 6.000, and a hydroxyl functionality from 2 to 6, more particularly from 3 to 5. In an embodiment, the polyether polyol is a random or blocked propylene oxide-ethylene oxide copolymer with or without ethylene oxide terminal (cap) groups. In another embodiment, the polyether polyol is a propylene oxide polymer with ethylene oxide terminal groups. In an embodiment, the polyol is a glycerol initiated polyether polyol built up of propylene oxide and ethylene oxide units and having a number average molecular weight from 2.000 to 6.000. Particularly, said polyol comprises 5-30 wt% of ethylene oxide. In an embodiment, the polyol is a propylene oxide adduct of glycerine containing of about 12 wt% random ethylene oxide with a hydroxyl number of about 55. In another embodiment, the polyol is a propylene oxide adduct of glycerine containing an end-capping of about 19 wt% of ethylene oxide, and with hydroxyl number of about 35.

In an embodiment, the polyol (b1) is a "polyester polyol". Suitable polyester polyols that can be used as polyol include those obtained from the condensation of one or more polyhydric alcohols, especially di- or trihydric alcohols, having from 2 to 15 carbon atoms with one or more polycarboxylic acids, especially dicarboxylic acids, having from 2 to 14 carbon atoms. Examples of suitable polyhydric alcohols include 1,2-ethanediol, ethylene glycol, propylene glycol such as 1,2-ethanediol, diethylene glycol, 1,2-propylene glycol and 1,3-propylene glycol, glycerol, neopentyl glycol, pentaerythritol, trimethylolpropane, 1,4,6-octanetriol, 1,4-butanediol, 1,5-pentanediol, 1,6-hexanediol, dodecanediol, octanediol, chloropentanediol, glycerol monallyl ether, glycerol monoethyl ether, diethylene glycol, 2-ethylhexanediol, 1,4-cyclohexanediol, 1,1,1-trimethylolpropane, 1,2,6-hexanetriol, 1,3,5-hexanetriol, 1,3-bis-(2-hydroxyethoxy)propane and the like. Examples of polycarboxylic acids include phthalic acid, isophthalic acid, terephthalic acid, tetrachlorophthalic acid, maleic acid, dodecylmaleic acid, octadecenylmaleic acid, fumaric acid, aconitic acid, trimellitic acid, 3,3'-thiodipropionic acid, succinic acid, adipic acid, trimethyladipic acid, malonic acid, glutaric acid, pimelic acid, sebacic acid, suberic acid, azelaic acid, maleic acid, fumaric acid, trimellitic acid, dodecanedicarboxylic acid, cyclohexane-1,2-dicarboxylic acid, 1,4-cyclohexadiene-1,2-dicarboxylic acid, 3-methyl-3,5-cyclohexadiene-1,2-dicarboxylic acid and the corresponding acid anhydrides, acid chlorides and acid esters such as phthalic anhydride, phthaloyl chloride and the dimethyl ester of phthalic acid. Particularly, suitable are polyester polyols obtained from the condensation of a diol having from 2 to 15 carbon atoms with a dicarboxylic acid having from 2 to 14 carbon atoms.

In an embodiment, the polyol (b1) is a "polycarbonate polyol". Suitable polycarbonate polyols that can be used as polyol include those obtained by reacting one or more polyhydric alcohols, especially di- or trihydric alcohols, having from 2 to 15 carbon atoms with one or more dialkyl carbonates, diaryl carbonates or phosgene. Examples of suitable polyhydric alcohols include 1,2-ethanediol, ethylene glycol, propylene glycol such as 1,2-ethanediol, diethylene glycol, 1,2-propylene glycol and 1,3-propylene glycol, glycerol, neopentyl glycol, pentaerythritol, trimethylolpropane, 1,4,6-octanetriol, 1,4-butanediol, 1,5-pentanediol, 1,6-hexanediol, dodecanediol, octanediol, chloropentanediol, glycerol monallyl ether, glycerol monoethyl ether, diethylene glycol, 2-ethylhexanediol, 1,4-cyclohexanediol, 1,1,1-trimethylolpropane, 1,2,6-hexanetriol, 1,3,5-hexanetriol, 1,3-bis-(2-hydroxyethoxy)propane and the like. Examples of suitable dialkyl carbonates and diaryl carbonates include dialkyl carbonates, diaryl carbonates having from 2 to 14 carbon atoms, such as dimethyl carbonate, diethyl carbonate, di-n-butyl carbonate, diphenyl carbonate and the like. Particularly suitable are polycarbonate polyols obtained from the condensation of a diol having from 2 to 15 carbon atoms with a dialkyl carbonate or diaryl carbonates having from 2 to 14 carbon atoms, or with phosgene.

In an embodiment, the polyol (b1) is a "polyether polycarbonate polyol". Suitable polyether polycarbonate polyols that can be used as polyols include those obtained by catalytic copolymerization of one or more alkylene oxides with carbon dioxide; particularly with a double metal cyanide (DMC) catalyst. Other suitable polyether polycarbonate polyols are those obtained by copolymerizing one or more H-functional initiator substances, one or more alkylene oxides and carbon dioxide; particularly in the presence of a double metal cyanide catalyst obtainable as defined above. Examples of suitable alkylene oxides include, among others, alkylenes having from 2 to 24 carbon atoms. Particularly, suitable alkylenes are those selected from the group consisting of ethylene oxide, propylene oxide, butene oxides, pentene oxides, hexene oxides, heptene oxides, octene oxides, nonene oxides, decene oxide, undecene oxides, dodecene oxides, cyclopentene oxide, cyclohexane oxide, cycloheptene oxide, cyclooctene oxide and styrene oxide. The above mentioned alkylene can be optionally substituted, such as alkylene oxides substituted with one mor more C₁-C₆ alkyl groups, preferably methyl or ethyl. Examples of more suitable alkylene oxides are selected from the group consisting of ethylene oxide, propylene oxide, butene oxide, styrene oxide and mixtures thereof. In a particular embodiment, the alkylene oxide is propylene oxide. The term "H-functional initiator substance" refers to a compound having H atoms active for the alkoxylation, such as, for example, alcohols, primary or secondary amines, or carboxylic acids. Examples of suitable H-functional initiator substances include, among others, one or more compounds selected from the group consisting of mono- or poly-hydric alcohols, polyvalent amines, polyvalent thiols, amino alcohols, thioalcohols, hydroxy esters, polyether polyols, polyester polyols, polyester ether polyols, polyether carbonate polyols, polycarbonate polyols, polycarbonates, polyethyleneimines, polyether amines, polytetrahydrofurans, polytetrahydrofuranamines, polyether thiols, polyacrylate polyols, castor oil, the mono- or di-glyceride of ricinoleic acid, monoglycerides of fatty acids, chemically modified mono-, di- and/or tri-glycerides of fatty acids, and C₁-C₂₄-alkyl fatty acid esters that contain on average at least 2 OH groups per molecule.

For the purpose of the present invention, all the aspects and embodiments (taken them alone or in combination with other embodiments disclosed above or below) disclosed in the invention with any one of the polyols taken separately also forms part of the invention.

The polymer polyol comprises polymeric particles comprising monomeric units of styrene, acrylonitrile, and alpha-methyl styrene and further one or more monomeric units selected from the group consisting of propenyl benzene; divinylbenzene; phenylacetylene; methylbutenes; methylpentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene and isomers thereof; and 2,4,6-triphenyl-1-hexene and isomers thereof.

For the purpose of the invention, the term "xylene" encompasses the ortho-, meta-, and para- isomers, unless specifically described. Further, for the purpose of the invention, wherein the stereo- and regio-selectivity of a term (compound) is not specifically disclosed, it is understood that this term encompasses all stereo- and regio-isomers. For example, the terms methyl-styrene and ethyl-styrene encompass all their isomers including the alpha-isomer, the cis-beta-isomer, the trans-beta-isomer, the ortho-isomer, the meta-isomer, and the para-isomer, unless specifically described. Then, for the purpose of the invention, the term "methyl-styrene" encompasses the prop-1-en-2-yl-benzene (alpha-methyl-styrene), 1-methyl-2-vinylbenzene (ortho-methyl-styrene), 1-methyl-3-vinylbenzene (meta-methyl-styrene), 1-methyl-4-vinylbenzene (para-methyl-styrene), (E)-prop-1-enylbenzene (trans-beta-methyl-styrene), and (Z)-prop-1-enylbenzene (cis-beta-methyl-styrene).

The terms of alkanes, alkenes and alkynes that are indicated in plural in the present invention indicate that they encompass all the possible regio- and stereo- isomers thereof. For example the term pentenes encompass 1-pentene, 2-cis-pentene, and 2-trans-pentene; and the term methyl butene encompasses 2-methyl-2-butene, 2-methyl-1-butene and 3-methyl-1-butene.

In an embodiment, the polymer polyol comprises from 10 to 65% by weight of polymeric particles in relation to the total weight of the polymer polyol measured by precipitation and separation method (see section 1.2.1. of the experimental data). For the purpose of the invention, the "content of polymeric particles in relation to the total weight of the polymer polyol" and the term "solid content" of the polymer polyol have the same meaning and they are used interchangeable.

In an embodiment, the polymeric particles (a1) comprise from 20 to 80 % by weight of monomeric units of styrene in relation to the total weight of the polymeric particles. In an embodiment, the polymeric particles (a1) comprise from 14 to 50 % by weight of monomeric units of acrylonitrile in relation to the total weight of the polymeric particles. In an embodiment, the polymeric particles (a1) comprise from 2 to 40 % by weight of monomeric units of alpha-methyl styrene in relation to the total weight of the polymeric particles. In an embodiment, the polymeric particles (a1) comprise from 0.1 to 10% by weight of one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene and isomers thereof; and 2,4,6-triphenyl-1-hexene and isomers thereof in relation to the total weight of the polymeric particles.

In an embodiment, the polymeric particles (a1) comprise:
from 20 to 80% by weight of styrene in relation to the total weight of the polymeric particles;
from 14 to 50 % by weight of acrylonitrile in relation to the total weight of the polymeric particles;
from 2 to 40 % by weight of alpha-methyl styrene in relation to the total weight of the polymeric particles; and
from 0.1 to 10% by weight of one or more monomeric units selected from the group consisting of propenyl benzene; divinylbenzene; phenylacetylene; methyl butenes; methyl pentenes; isobutene; pentenes; hexene; heptenes; octenes, cyclopentadiene;
allylbenzene; methyl-styrene other than alpha-methyl-styrene; ethyl-styrene; stilbene; 2,4-diphenyl-1-butene and isomers thereof; 1,4-diphenyl-1,3-butadiene and isomers thereof; and 2,4,6-triphenyl-1-hexene and isomers thereof in relation to the total weight of the polymeric particles;
being the sum of the monomers up to 100% by weight of the polymeric particles.

In an embodiment, the polymeric particles (a1) comprise:
from 40 to 70 % by weight of styrene in relation to the total weight of the polymeric particles;
from 20 to 40 % by weight of acrylonitrile in relation to the total weight of the polymeric particles;
from 2 to 10 % by weight of alpha-methyl styrene in relation to the total weight of the polymeric particles;
from 0.1 to 3% by weight of one or more monomeric units selected from the group consisting of propenyl benzene; divinylbenzene; phenylacetylene; methyl butenes; methyl pentenes; isobutene; pentenes; hexene; heptenes; octenes, cyclopentadiene;
allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and isomers thereof in relation to the total weight of the polymeric particles;
being the sum of the monomers up to 100% by weight of the polymeric particles.

In an embodiment, the polymeric particles (a1) comprise:
from 60 to 70 % by weight of styrene in relation to the total weight of the polymeric particles;
from 25 to 38 % by weight of acrylonitrile in relation to the total weight of the polymeric particles;
from 2 to 5 % by weight of alpha-methyl styrene in relation to the total weight of the polymeric particles;
from 0.1to 2.5% by weight of one or more monomeric units selected from the group consisting of propenyl benzene; divinylbenzene; phenylacetylene; methyl butenes; methyl pentenes; isobutene; pentenes; hexene; heptenes; octenes, cyclopentadiene;
allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and isomers thereof in relation to the total weight of the polymeric particles;
being the sum of the monomers up to 100% by weight of the polymeric particles.

In an embodiment, the polymeric particles (a1) comprise:
from 63 to 66 % by weight of styrene in relation to the total weight of the polymeric particles;
from 30 to 34 % by weight of acrylonitrile in relation to the total weight of the polymeric particles;
from 2 to 3 % by weight of alpha-methyl styrene in relation to the total weight of the polymeric particles;
from 0.1 to 2% by weight of one or more monomeric units selected from the group consisting of propenyl benzene; divinylbenzene; phenylacetylene; methyl butenes; methyl pentenes; isobutene; pentenes; hexene; heptenes; octenes, cyclopentadiene;
allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and isomers thereof in relation to the total weight of the polymeric particles;
being the sum of the monomers up to 100% by weight of the polymeric particles.

In an embodiment, the polymer particles (a1) comprise a particle size from 0.1 to 10µm. The term "particle size" refers to the particle diameter of the dispersed particles assuming that the particles have a spherical or quasi-spherical shape. When referring to a set of polymer particles as being of a particular size, it is contemplated that the set of particles can have a distribution of sizes around the specified size. Thus, as used herein, a size of a set of particles can refer to a mode of a distribution of sizes, such as a peak size of the distribution of sizes (i.e. particle size distribution). The method for determining the particle size (PS) and particle size distribution (PSD) of the dispersed polymer particles can be any method disclosed in the state of the art. For the purpose of the invention the PS is measured by laser diffraction using Malvern Mastersizer 3000 Instrument (see section 1.2.3. of the experimental section).

In an embodiment, the polymer polyol comprises from 35 to 90% by weight of the polyol (b1) in relation to the total weight of the polymer polyol.

In an embodiment, the polymer polyol has a dynamic viscosity from 500 cp to 30000 cp measured according to the standard EN ISO 3219 guidelines (see section 1.2.2. of the experimental data). In an embodiment, the polymer polyol has a dynamic viscosity from 3500 cp and 25000cp measured according to the standard EN ISO 3219 guidelines. In an embodiment, the polymer polyol wherein the content of polymeric particles in relation to the total weight of the polymer polyol is from 55 to 65% of and from 19000 cp to 21000 cp measured according to the standard EN ISO 3219 guidelines. In an embodiment, the polymer polyol wherein the content of polymeric particles in relation to the total weight of the polymer polyol is from 40 to 50% of and from 3000 cp to 4500 cp measured according to the standard EN ISO 3219 guidelines.

A polymer polyol as defined in the first aspect of the invention obtainable by the process of the second aspect of the invention as defined herein below is also part of the invention. All the embodiments disclosed above for the polymer polyol, including the polymeric particles and the continuous polyether polyol phase of the first aspect of the invention as well as all the embodiments disclosed above for the process for their preparation of the second aspect of the invention including conditions and reagents also apply for the polymer polyol obtainable by this process. For the purpose of the invention, the expressions "obtainable", "obtained" and equivalent expressions are used interchangeably, and in any case, the expression "obtainable" encompasses the expression "obtained".

As it is disclosed above, the second aspect of the invention relates to a process for the preparation of a polymer polyol comprising the use of pyrolysis oil of polystyrene. Particularly to a process for the preparation of a polymer polyol comprising:
(a) a dispersed phase formed by polymeric particles (a1); and
(b) a continuous phase comprising a polyol (b1);
wherein:
the polymeric particles (a1) comprise:
monomeric units of styrene, acrylonitrile, and alpha-methyl styrene; and
one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and isomers thereof; and
wherein the process comprises:
   (i) providing polymeric particles dispersed in a continuous polyol phase by polymerization of a polystyrene pyrolysis oil, and acrylonitrile; or alternatively, by polymerization of a polystyrene pyrolysis oil, acrylonitrile, styrene monomer; and one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene and isomers thereof; and 2,4,6-triphenyl-1-hexene and isomers thereof;
in a continuous polyol phase in the presence of at least one radical initiator; wherein the polystyrene pyrolysis oil comprises from 50% to 98% by weight of styrene monomer.

In an embodiment, the process of the second aspect of the invention further comprises an additional previous step (ii) before step (i) which comprises providing a polystyrene pyrolysis oil comprising from 50% to 98% by weight of styrene monomer by submitting one or more polystyrene compounds and/or one or more polystyrene containing compounds to a pyrolysis reaction (for instance see US10301235, WO2021230312 and WO2021053074).

The term "polystyrene" refers to a polymer made from the styrene monomer (SM; or C₆H₅CH=CH₂) of formula:

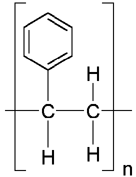

For the purpose of the invention, the term polystyrene encompasses, in all their grades, polystyrene, polymers and copolymers comprising styrene, and mixture thereof. In an embodiment, the polystyrene is a residue (waste) containing polystyrene, styrene, or mixtures thereof. Examples of suitable polymers containing styrene for the present invention includes, but it is not limited to, High Impact Polystyrene (HIPS), styrene acrylonitrile (SAN), Acrylonitrile Butadiene Styrene (ABS), General Purpose polystyrene (GPPS), Expanded polystyrene foam (EPS).

For the purpose of the invention, the term "pyrolysis oil of polystyrene", "polystyrene pyrolysis oil" and "pyrolysis oil" have the same meaning and are used interchangeable. They refer to an oil mixture containing from 50% to 98% of styrene monomer obtainable by the pyrolysis of polystyrene as defined above. Pyrolysis is a well-established technique for decomposition of organic material into oil and other constituents at elevated temperatures (particularly, above its decomposition temperature), in an inert atmosphere (in the absence of oxygen). Appropriate pyrolysis oils for the present invention and processes for their preparation are widely disclosed in the state of the art. Examples can be found in Ki-bum Park et al. "Two-stage pyrolysis of polystyrene: Pyrolysis oil as a source of fuel or benzene, toluene, ethylbenzene, and xylenes". Applied Energy 259, 2020, pp. 114240; Ibrahim M. Maafa. "Pyrolysis of Polystyrene Waste: A review". Polymers, 2021, vol. 13, pp. 225.; John Scheirs, and Walter Kaminsky. "Feedstock Recycling and Pyrolysis of Waste Plastics: Converting Waste Plastics into Diesel and Other Fuels", Wiley Online Library, 2006, p.635; Jasmin Shah et al. "Conversion of waste polystyrene through catalytic degradation into valuable products", Korean J. Chem. Eng., 2014, vol. 31(8), pp.1389-1398; US10301235; WO2021230312; and WO2021053074.

In an embodiment, the process is one wherein the pyrolysis oil comprises from 60 to 98% by weight of styrene monomer. In an embodiment, the process is one wherein the pyrolysis oil comprises from 80 to 98% by weight of styrene monomer. In an embodiment, the process is one wherein the pyrolysis oil comprises from 80 to 95% by weight of styrene monomer.

In an embodiment, the process is one wherein the pyrolysis oil comprises:
from 50 to 98% by weight of styrene monomer;
from 0.1 to 10% by weight of alpha-methyl styrene;
from 0.0005 to 0.1% by weight of phenylacetylene;
from 0.2 to 15% by weight of toluene;
from 0 to 5% by weight of benzene;
from 0.5 to 12% by weight of C₈ aromatic compounds;
from 0.1 to 3% by weight of C₉ aromatic compounds;
from 0.01 to 1% by weight of C₁₀ aromatic compounds; particularly selected from the group consisting of divinylbenzene, ethyl-styrene and a mixture thereof;
from 0 to 1% by weight of a (C₃-C₈)fraction comprising one or more compounds selected form the group consisting of (C₃-C₈)alkanes, (C₃-C₈)alkenes, (C₅-C₈)cycloalkanes and (C₅-C₈)cycloalkenes;
from 0 to 15% by weight of a heavy oil fraction; and
from 0.5 to 5000ppm of heteroatom containing compounds

In an embodiment, the process is one wherein the pyrolysis oil comprises:
from 50 to 98% by weight of styrene monomer;
from 0.1 to 10% by weight of alpha-methyl styrene;
from 0.001 to 0.1% by weight of phenylacetylene;
from 0.2 to 15% by weight of toluene;
from 0 to 5% by weight of benzene;
from 0.5 to 12% by weight of C₈ aromatic compounds comprising ethylbenzene; ortho-xylene, meta-xylene, and para-xylene;
from 0.1 to 3% by weight of C₉ aromatic compounds comprising cumene; propenylbenzene, n-propylbenzene; allyl-benzene, ethyl-toluene, and methyl-styrene other than alpha-methyl styrene;
from 0.01 to 1% by weight of C₁₀ aromatic compounds; particularly selected from the group consisting of divinylbenzene, ethyl-styrene and a mixture thereof;
from 0 to 1% by weight of a (C₃-C₈)fraction comprising one or more compounds selected form the group consisting of (C₃-C₈) alkanes, (C₃-C₈) alkenes, (C₅-C₈)cycloalkanes and (C₅-C₈)cycloalkenes;
from 0 to 15% by weight of a heavy oil fraction; and
from 0.5 to 5000ppm of heteroatom containing compounds.

In an embodiment, the process is one wherein the pyrolysis oil comprises:
from 60 to 98% by weight of styrene monomer;
from 1 to 5% by weight of alpha-methyl styrene;
from 0.001 to 0.05% by weight of phenylacetylene;
from 0.5 to 5% by weight of toluene;
from 0 to 3% by weight of benzene;
from 1 to 6% by weight of C₈ aromatic compounds; particularly comprising ethylbenzene; ortho-xylene, meta-xylene, and para-xylene;
from 0.1 to 2% by weight of C₉ aromatic compounds; particularly comprising cumene, propenylbenzene, n-propylbenzene, allyl-benzene, ethyl-toluene, and methyl-styrene other than alpha-methyl-styrene;
from 0.01 to 1% by weight of C₁₀ aromatic compounds; particularly selected from the group consisting of divinylbenzene, ethyl-styrene and a mixture thereof;
from 0 to 0.5% by weight of a (C₃-C₈)fraction comprising one or more compounds selected form the group consisting of (C₃-C₈) alkanes, (C₃-C₈) alkenes, (C₅-C₈)cycloalkanes and (C₅-C₈)cycloalkenes;
from 0 to 10% by weight of a heavy oil fraction; and
from 10 to 500ppm of heteroatom containing compounds

In an embodiment, the process is one wherein the pyrolysis oil comprises:
from 70 to 98% by weight of styrene monomer;
from 1 to 3.5% by weight of alpha-methyl styrene;
from 0.001 to 0.005% by weight of phenylacetylene;
from 1 to 3% by weight of toluene;
from 0 to 0.0001% by weight of benzene;
from 1 to 4.5% by weight of C8 aromatic compounds; particularly comprising ethylbenzene; ortho-xylene, meta-xylene, and para-xylene;
from 0.1 to 1% by weight of C₉ aromatic compounds; particularly comprising cumene, propenylbenzene, n-propylbenzene, allyl-benzene, ethyl-toluene, and methyl-styrene other than alpha-methyl-styrene;
from 0.01 to 1% by weight of C₁₀ aromatic compounds; particularly selected from the group consisting of divinylbenzene, ethyl-styrene and a mixture thereof;
from 0 to 0.3% by weight of a (C₃-C₈)fraction comprising one or more compounds selected form the group consisting of (C₃-C₈) alkanes, (C₃-C₈) alkenes, (C₅-C₈)cycloalkanes and (C₅-C₈)cycloalkenes;
from 0 to 10% by weight of a heavy oil fraction; and
from 10 to 500ppm of heteroatom containing compounds

In an embodiment, the process is one wherein the pyrolysis oil as defined above further comprises: from 0.005 to 1 mg KOH/g of acid containing compounds measured by the standard test method ASTM D664; and from 0.1 to 50 ppm of metals measured by the method EPA 3051a and ASTM D6052-97.

The term "alkane" refers to a saturated straight hydrocarbon, optionally branched, which contains the number of carbon atoms specified in the description or claims. Examples include n-butane, isobutane, pentane, hexane, methylbutane, heptane and octane.

The term "cycloalkane" refers to a saturated cycle hydrocarbon, optionally branched, which contains the number of carbon atoms specified in the description or claims. Examples include cyclopentane and cyclohexane.

The term "alkene" refers to a straight hydrocarbon, optionally branched, which contains the number of carbon atoms specified in the description or claims, and that also contains at least one double bond. Examples include 2-methyl-1-butene, pentene, heptene, hexene, and isobutene.

The term "cycloalkene" refers to a cycle hydrocarbon, optionally branched, which contains the number of carbon atoms specified in the description or claims, and that also contains at least one double bond. Examples include cyclopentadiene.

The term "aromatic compound" refers to those organic compounds that contain one or more rings with pi electrons delocalized all the way around them which contains the number of carbon atoms specified in the description or claims. The aromatic compounds can be optionally substituted by one or more moieties such as for examples (C₁-C₆)alkyl, (C₂-C₆)alkenyl and (C₂-C₆)alkynyl. In an embodiment, the aromatic compound is a phenyl optionally substituted by (C₁-C₆)alkyl, (C₂-C₆)alkenyl, and (C₂-C₆)alkynyl. The term "alkyl" refers to a saturated straight hydrocarbon substituent, optionally branched, which contains the number of carbon atoms specified in the description or claims. Examples include, among others, the group methyl, ethyl, propyl, isopropyl, butyl, isobutyl, sec-butyl, and tert-butyl. The term "alkenyl" refers to straight hydrocarbon substituent, optionally branched, which contains the number of carbon atoms specified in the description or claims, and that also contains at least one double bond. The term "alkynyl" refers to straight hydrocarbon substituent, optionally branched, which contains the number of carbon atoms specified in the description or claims, and that also contains at least one triple bond. In an embodiment, the aromatic compounds are selected from the group consisting of C₈ aromatic compounds, C₉ aromatic compounds and C₁₀ aromatic compounds. Examples of C₈ aromatic compounds include ethylbenzene; xylenes (dimethyl benzene) such as ortho-xylene, meta-xylene, and para-xylene; and phenylacetylene. Examples of C₉ aromatic compounds include cumene (isopropyl benzene), propenyl benzene, n-propyl benzene, allyl-benzene, ethyl-toluene, and methyl-styrene. Examples of C₁₀ aromatic compounds include divinylbenzene and ethyl-styrene.

The term "heavy oil fraction" refers to a mixture with an initial normal boiling point of 200°C and final normal boiling point of 400°C. It encompasses one or more of the following compounds indene, naphthalene, tetrahydronaphthalene, 2,4-diphenyl-1-butene, 1,4-diphenyl-1,3-butadiene, 2,4,6-triphenyl-1-hexene, biphenyl, bibenzyl, methylnaphthalene, 2-phenylnaphtalene, diphenylmethane, and stilbene among others.

The term "acid containing compounds" refers to compounds comprising one or more acids moieties. It encompasses both organic carboxyl acids and inorganic acids. Examples include, among others, hydrochloric acid, hydrobromic acid, benzoic acid, acetic acid, and propionic acid.

The term "metal" encompasses for instance Calcium, Potassium, Silicium, aluminium, sodium, magnesium, Titanium, Iron, Zinc, among others.

The term "heteroatom" encompasses halogen (for instance chlorine, fluorine, and bromine), sulphur, nitrogen, and oxygen. And the term "heteroatom containing compounds" encompasses compounds that comprises at least one or more heteroatoms as defined above.

In an embodiment, the process comprises a polymerization process of styrene monomer, pyrolysis oil and acrylonitrile in the continuous polyol phase in the presence of at least one radical initiator. It means that the pyrolysis oil is partially substituting the styrene monomer. In an embodiment, the process comprises a polymerization of pyrolysis oil and acrylonitrile, in the continuous polyol phase in the presence of at least one radical initiator. It means that the pyrolysis oil is the only source of styrene monomer.

In an embodiment, the process is one wherein the polymer polyol thus obtained is the polymer polyol of the first aspect of the invention. All embodiments mentioned above for the polymer polyol also apply here for the process for the invention.

All the reaction conditions disclosed in the state of the art for the preparation of styrene based polymer polyols, and particularly SAN polymer polyols are appropriate for the process of the present invention. In particular, the appropriate reagents and their amounts (radical initiator, macromer, dispersant, initiator, chain transfer agent ...), the reaction conditions (temperature, time, addition rate...), as well as the type of process (batch, semibatch or continuous) and/or the use of seeding can readily be determined by those skilled in the art according to the field and the type and amount of polymer polyol being prepared.

The temperature range may vary from about 80°C to about 150° or perhaps greater, the preferred range being from about 90°C to about 140°C; more preferably from about 100°C to about 135°C, and most preferably from about 110 °C to about 130°C.

In an embodiment, the process of the invention is a batch process. In an embodiment, the process of the invention is a semi-batch process. In an embodiment, the process of the invention is a continuous process. In an embodiment, the process of the invention is a semi-batch process and further comprises the use of one or more macromers. In an embodiment, the process of the invention is a continuous process and further comprises the use of one or more macromers. In an embodiment, the process of the invention is a continuous process and further comprises the use of one or more preformed dispersant.

The term "macromer" or "macromonomer" refers to a molecule which comprises one or more polymerizable double bonds able to copolymerize with ethylenically unsaturated monomers such as styrene, acrylonitrile, and alpha methyl styrene and which may comprise one or more hydroxyl-terminated polyether chains. Examples of macromers appropriate for the present invention and processes for their preparation are well known in the art (see. US4454255). Typical macromers include polyols, preferably polyether polyols, having an unsaturated group, which are commonly manufactured by reacting a polyol with an organic compound containing an unsaturated group and a carboxyl, anhydride, isocyanate, epoxy, or other functional group able to react with active hydrogencontaining groups. In an embodiment, the macromer is prepared by the reaction of a polyol, either directly or indirectly (e.g., by previous reaction of the polyol with a diisocyanate, such as TDI), with an unsaturated compound. More specifically, an unsaturated compound selected from maleic anhydride, fumaric acid, dialkyl fumarates, dialkyl maleates, glycol fumarates, glycol maleates, 1,1-dimethyl-m-isopropenylbenzyl isocyanate, isocyanato ethyl methacrylate, isocyanato ethyl methacrylate, isocyanato ethyl methylmethacrylate, 2-butene-1,4-diol, 1-butene-3,4-diol, hydroxymethyl methacrylate, hydroxypropyl acrylate, hydroxyethyl methacrylate, methyl methacrylate, acrylic acid, methacrylic acid, acrylic anhydride, methacrylic anhydride, methacroyl chloride, glycidyl methacrylate and allyl glycidyl ether. In a preferred embodiment, the macromer is the reaction product of a polyol with maleic anhydride (MA) or 1,1-dimethyl-m-isopropenylbenzyl isocyanate (TMI). Optionally, the polyol used to prepare the macromer may be obtained by linkage of polyols through coupling with a compound such as a Poly isocyanate (e.g., tolylene diisocyanate (TDI), diphenylmethane-4,4'-diisocyanate (MDI), hexamethylene disisocyanate (HMDI)) to produce a high molecular weight polyol, which is then reacted with the unsaturated compound. In the preparation of the macromer, it is recommended that the quantity of the reactive unsaturated compound used is in the range from 0.3 to 2.5 moles per mol of polyol, and particularly from 0.5 to 2.0 moles per mol of polyol. In an embodiment, the reactive unsaturated compound used is maleic anhydride. In an embodiment, the reactive unsaturated compound used is 1,1-dimethyl-m-isopropenylbenzyl isocyanate. The polyol used for preparing the macromer is selected from the group consisting of polyether polyol, polyester polyol, polycarbonate polyol and a mixture thereof. Suitable polyether polyols, polyester polyols, and polycarbonate polyols for the preparation of the macromer can be the same disclosed herein above and below for the preparation of the polyol (b1). All mentioned above and below for the preparation of the polyol (b1) also apply here for the preparation of the macromer. In an embodiment, the polyol used for preparing the macromer is a polyether polyol. In an embodiment, the polyol used for preparing the macromer is selected from a polyoxyalkylene polyether polyol as defined herein above and below. In an embodiment, the polyol used for preparing the macromer is a polyether polyol obtained by polymerization of alkylene oxides having from 2 to 4 carbon atoms (e.g., ethylene oxide, propylene oxide, butylene oxide and mixtures thereof) in the presence of a starting compound having at least one active hydrogen atom, preferably 2-6 active hydrogen atoms. In an embodiment, the polyol used for preparing the macromer is a polyether polyol built up of propylene oxide and ethylene oxide units and preferably having a molecular weight from 1500 to 12000, and a hydroxyl functionality from 2 to 6, more preferably from 3 to 6. In an embodiment, the polyol used for preparing the macromer is a polyether polyol which is a random or blocked propylene oxide-ethylene oxide copolymer with or without ethylene oxide terminal (cap) groups. In an embodiment, the polyol used for preparing the macromer is a polyether polyol which is a propylene oxide polymer with ethylene oxide terminal groups. In an embodiment, the polyol is a glycerol or sorbitol-initiated polyether polyol built up of propylene oxide and ethylene oxide units and having a hydroxyl number from 20 to 45. In an embodiment, the polyol used for preparing the macromer comprises from 5 to30 % by weight of ethylene oxide; particularly from 10 to 20 t% by weight. In an embodiment, the polyol used for preparing the macromer is a propylene oxide adduct of sorbitol containing about 16 wt% ethylene oxide caps with hydroxyl number of about 29. In an embodiment, the polyol used for preparing the macromer is a propylene oxide adduct of glycerin containing of about 13 wt% ethylene oxide caps with hydroxyl number of about 35.

The terms "dispersant" and "stabilizer" have the same meaning and they are interchangeable. Dispersants or stabilizers are typically employed in the preparation of polymer polyols to stabilize the dispersion of polymers resulting from polymerization of the ethylenically unsaturated monomers in the polyol phase. In an embodiment, the stabilizer is generated "in situ" adding a macromer to the reaction mixture for preparing the polymer polyol. In an embodiment of the present invention, the stabilizer is previously prepared and then added to the reaction mixture; these stabilizers are commonly known as "pre-formed" stabilizer. The terms "pre-formed stabilizer" and "pre-formed dispersant" have the same meaning and they are interchangeable. They refer to an intermediate compound obtained by reacting a macromer (as defined herein below and above) with at least one ethylenically unsaturated monomer (e.g., acrylonitrile, styrene, methylmethacrylate, etc.), optionally with a chain transfer agent and optionally in the present of a diluent (for instance methanol, isopropanol, toluene, ethylbenzene, polyether polyols, among others) to give a copolymer dispersion having commonly a low solids content (e.g., lower than 20% by weight, such as for example from 3 to 15 % by weight). The resulting dispersant allows the stabilization of the solid particles of the polymer polyol. Pre-formed stabilizers and processes for their preparation are well known in the art.

Suitable chain transfers agents for use in the practice of the present invention include, but it is not limited to, isopropanol, ethanol, tert-butanol, methanol, toluene, ethylbenzene, trimethylamine, water, cyclohexane, terpinolene, mercaptans such as dodecanethiol, ethanethiol, 1-heptanethiol, 2-octanethiol and toluenethiol. In an embodiment, the chain transfer agent is terpinolene.

Examples of appropriate (free-radical polymerization) initiators include but it is not limited to peroxides including both alkyl and aryl hydroperoxides, acyl peroxides, peroxy-esters, persulfates, perborates, percarbonates and azo compounds, for example, hydrogen peroxide, dibenzoyl peroxide, didecanoyl peroxide, lauroyl peroxide, t-butyl hydroperoxide, benzoyl peroxide, di-t-butyl peroxide, di(3,5,5-trimethylhexanoyl)peroxide, t-butyl-peroxy diethyl acetate, t-butyl peroctoate, t-butyl peroxy isobutyrate, t-butyl peroxy 3,5,5-trimethyl hexanoate, t-butyl perbenzoate, t-butyl peroxy pivalate, t-butyl peroxy-2-ethyl hexanoate, tert-amyl peroxy-2-ethylhexanoate, (1,1,3,3-tetramethylbutyl peroxy-2-ethylhexanoate), cumene hydroperoxide, azobis(isobutyronitrile) (AIBN) and 2,2'-azo bis-(2-methylbutyronitrile) (AMBN), and mixtures thereof. In an embodiment, the initiator is 1,1,3,3-tetramethylbutyl peroxy-2-ethylhexanoate. In another embodiment, the initiator is tert-amyl peroxy-2-ethylhexanoate. In another embodiment, the initiator is di(3,5,5-trimethylhexanoyl)peroxide.

In an embodiment, the process of the invention is performed in the present of a solvent (also known as diluents). Examples of suitable solvents for the present invention include, but it is not limited to, mono-alcohols (i.e., monohydroxy alcohols), polyols, hydrocarbons, ethers, and mixtures thereof. Suitable mono-alcohols include all alcohols which contain from 1 to 4 carbon atoms such as, for example, methanol, ethanol, n-propanol, isopropanol, n-butanol, sec.-butanol, tert-butanol, n-pentanol, 2-pentanol, 3-pentanol, and mixtures thereof.

In an embodiment, the process further comprises an additional step of recovering the unreacted monomers (if any) and/or chain transfer agent and they subsequent use as starting material. Said recovery is usually carried out by means of vacuum evaporation of unreacted monomers and other vaporizable compounds contained in the reaction product and subsequent vapor product condensation. Means for vaporization are those typical of the state of the art, such as flash evaporation, stripping, and distillation.

In an embodiment, the process further comprises an additional step of purifying or fractionating the polymer polyol thus obtained. This step is commonly performed by means of vacuum stripping using an inert gas and/or steam or thin film vacuum distillation or short path vacuum distillation.

In an embodiment, the process further comprises an additional step of volatile organic compounds (VOCs) treatment (i.e., VOCs eliminating). This treatment is typically performed by the use of scrubbers or thermal oxidating agents.

As it is mentioned above, polymer polyols are commercial products that have found use in a variety of applications. One of their main applications is as raw material for the preparation of polyurethane foams such as polyurethane rigid and flexible foams. Therefore, it is part of the invention the use of a polymer polyol of the first aspect of the invention for the production of polyurethane foams. The primary function of polymer polyols is to enhance the hardness or stiffness of the polyurethane and, in particular, to enhance the load bearing or energy adsorbing capacity of polyurethane foams. End-use applications of polyurethane foams include, for example, mattresses, furniture, carpet pad; packaging and energy management, thermal insulation, and automotive seating, trim, headliners, sound insulation, crash pads, etc.

Other applications for polymer polyols are the so-called CASE applications, the acronym for coatings, adhesives, sealants, and elastomers, which can be present in coating materials or varnishes, adhesives, vehicle coatings, cables, floorings, walls, and roads among others.

Other aspect of the present invention relates to compositions comprising the polymer polyol of the first aspect of the invention together with one or more excipients or carriers. It is also part of the invention compositions made from the polymer polyol of the first aspect of the invention, optionally comprising one or more excipients or carriers.

The process for the preparation of the above-mentioned compositions as well as the type of excipients and/or carriers and their amounts can readily be determined by those skilled in the art according to the type and purpose of the composition being prepared.

In an embodiment, the composition is a polyurethane made from the polymer polyol of the present invention; particularly a polyurethane based foam. Thus, the present invention also relates to the use of the polymer polyol of the present of the invention for the preparation of polyurethane such as polyurethane foams.

In an embodiment, the composition is a polyurethane foam made from the polymer polyol of the invention; particularly obtainable by a process comprising reacting the polymer polyol with at least one di- or poly-isocyanate; and at least a blowing agent. Further, a process for the preparation of a polyurethane, comprising the reaction of at least one polymer polyol as defined in the first aspect of the invention with at least one di- or poly-isocyanate; and at least a blowing agent is also part of the invention.

For example, it can be performed in the presence of a radical initiator. Examples of suitable blowing agents include water, liquid CO₂, and methylene chloride. Optionally, the process can further comprise the use of additional compound such as surfactant. The appropriate starting materials and reagents as well as their amounts and the specific reaction conditions (such as the type of process being used- batch, semibatch or continuous) can readily be determined by the skilled in the art regarding the type of polyurethane foam being prepared (for instance flexible or rigid polyurethane foam).

As it is demonstrated in the experimental section, the polyurethane foams obtained by the use of polymer polyol of the present invention (Examples 1 and 2) which has been prepared using polystyrene pyrolysis oil as the unique source of styrene monomer has comparable values, and therefore properties, as those foams obtained using "pure" styrene monomer (Foam Comparative Ex. 3). Therefore, it is concluded that the use of pyrolysis oil does surprisingly neither affect the foaming process, nor the properties of the polyurethane foams obtained from the polymer polyol of the present invention, even when the pyrolysis oil used does not required further fractionating/purifying steps.

In an embodiment, the polyurethane foam made from the polymer polyol of the present invention is characterized by having at least one of the following properties:
Density from 50 to 60 kg/m3 measured by ASTM D3574
C.L.D. 40% from 12 to 15 kPa measured by ISO 3386-1;
Resilience greater to 35% measured by ASTM D3574;
Compression set 75% from less than 4,0% measured by ISO 1856; and
Permeability from 500 to 800 mm/s measured by ISO 9237.

In an embodiment, the polyurethane foam made from the polymer polyol of the present invention is characterized by having at least two of the following properties:
Density from 50 to 60 kg/m3 measured by ASTM D3574
C.L.D. 40% from 12 to 15 kPa measured by ISO 3386-1;
Resilience greater to 35% measured by ASTM D3574;
Compression set 75% from less than 4,0% measured by ISO 1856; and
Permeability from 500 to 800 mm/s measured by ISO 9237.

In an embodiment, the polyurethane foam made from the polymer polyol of the present invention is characterized by having at least three of the following properties:
Density from 50 to 60 kg/m3 measured by ASTM D3574
C.L.D. 40% from 12 to 15 kPa measured by ISO 3386-1;
Resilience greater to 35% measured by ASTM D3574;
Compression set 75% from less than 4,0% measured by ISO 1856; and
Permeability from 500 to 800 mm/s measured by ISO 9237.

In an embodiment, the polyurethane foam made from the polymer polyol of the present invention is characterized by having the following properties:
Density from 50 to 60 kg/m3 measured by ASTM D3574
C.L.D. 40% from 12 to 15 kPa measured by ISO 3386-1;
Resilience greater to 35% measured by ASTM D3574;
Compression set 75% from less than 4,0% measured by ISO 1856; and
Permeability from 500 to 800 mm/s measured by ISO 9237.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The present invention covers all possible combinations of particular and preferred embodiments described herein.

### Examples

### 1. Polymer polyol

### 1.1. Reagents

Polyol: a propylene oxide adducts of glycerin containing about 12 % by weight of random ethylene oxide with a hydroxyl number of about 55 and a viscosity of 490 mPa·s (Alcupol^{®} F-5511 available from Repsol Quimica).
Chain transfer agent: Terpinolene available from Merck

### Monomers:

- Styrene Monomer (SM) having more than 99% purity available from Merck
- Acrylonitrile Monomer (can) having more than 99% purity available from Merck

### Pyrolysis oil

The pyrolysis oils used as starting materials having the composition as disclosed in the present application are summarized herein below:
- polystyrene pyrolysis oil (pyrolysis oil 1) is an oil obtained by pyrolysis of polystyrene having at least 50% of SM (see US10301235, WO2021230312 and WO2021053074 having a composition typo as disclosed below, and which is not further purified).
- Fractionated polystyrene pyrolysis oil (pyrolysis oil 2) is the oil obtained after eliminating the light and heavy fractions of the polystyrene pyrolysis oil 1 (see the semibatch synthesis of polymer polyol of Ex. 2 section).

The content of the main components of the polystyrene pyrolysis oil 1 and the fractionated polystyrene pyrolysis oil 2 expressed in % by weight are listed herein below:

| Components | polystyrene pyrolysis oil 1 | Fractionated polystyrene pyrolysis oil (pyrolysis oil 2) |
|---|---|---|
| Styrene monomer (SM) | 82 | 92 |
| Ethyl benzene (EB)^{(d)} | 2.3 | 3.60 |
| N-propyl benzene (NPBz^{)(e)} | 0.10 | 0.16 |
| Cumene^{(e)} | 0.30 | 0.47 |
| Alpha methyl styrene (AMS^{)(e)} | 2.9 | 3 |
| Toluene | 4.6 | 0.26 |
| benzene | 0.30 | 30 PPM |
| xylenes^{(d)} | 0.30 | 0.47 |
| Heavy oil fraction ^{(a)} | 6 | < 20 PPM |
| C₃-C₈ fraction ^{(b)} | 0.2 | < 10 PPM |
| Other C₈-C₁₀ Aromatics^{(c)} | 1 | 0.05 |

| | | |
|---|---|---|
| (a) including among others indene, naphthalene, tetrahydronaphthalene, 2,4-diphenyl-1-butene, 1,4-diphenyl-1,3-butadiene, 2,4,6-triphenyl-1-hexene, biphenyl, bibenzyl, methylnaphthalene, 2-phenylnaphtalene, diphenylmethane, and stilbene. (b) C₃-C₈ fraction refers to the fraction comprising one or more compounds selected form the group consisting of (C₃-C₈)alkanes, (C₃-C₈)alkenes, (C₅-C₈)cycloalkanes and (C₅-C₈)cycloalkenes; (c) including the C10 aromatic compounds; and other C8 and C9 aromatic compounds than those specifically disclosed in the Table, such as ethyl-toluene, methyl-styrene other than alpha-methyl-styrene, ethyl-styrene, allyl-benzene, divinylbenzene, phenylacetylene among others (d) corresponding to C8 aromatic compounds (e) corresponding to C9 aromatic compounds | | |

### Initiators:

- Initiator A (Trigonox 421): 1,1,3,3-tetramethylbutyl peroxy-2-ethylhexanoate (available from Nouryon)
- initiator B (Peroxan APO): tert-amyl peroxy-2-ethylhexanoate (available from Pergan)

### Macromer:

Macromer A: a propylene oxide adducts of sorbitol containing 16 % by weight of ethylene oxide cap with hydroxyl number of 29 (polyol). This macromer is prepared by reacting, under heating at 90°C, the polyol with 0,9 moles of isopropenyl dimethyl benzyl isocyanate (sold as TMI^{®} (META) by Allnex) per mole of polyol in the presence of 300 ppmw of tin(II) 2-ethylhexanoate as catalyst for 3 hours under nitrogen atmosphere, resulting in a molecule containing polymerizable carbon-carbon double bonds.

Macromer B: it was prepared by heating a propylene oxide adduct of glycerine containing 13 % by weight of ethylene oxide cap with hydroxyl number of 35 (polyol) with 1.6 parts by weight of maleic anhydride per part of polyol and 0.01 parts by weight of calcium (II) 2-ethylhexanoate catalyst per part of polyol at 145°C for about 1 hour in a nitrogen atmosphere obtaining and intermediate product. This intermediate product was subsequently reacted with 0.06 parts by weight of propylene oxide per part of polyol at 145°C for 4 hours. Volatiles were stripped off at 110°C under vacuum using nitrogen as stripping gas, resulting in a molecule containing 0.75 polymerizable carbon-carbon double bonds per mole of polyol.

### Catalyst

Niax^{®} DMEA and Niax^{®} Catalyst A-107 are amine catalysts available from Momentive
Kosmos^{®}29 is stannous octoate-based metal catalyst available from Evonik

### Surfactant

Tegostab^{®} B8002 is a silicone based surfactant available from Evonik

### Polyurethane starting material

TDI T80/20 is Desmodur^{®} T80 is a mixture of the two isomeric forms 2,4-toluene diisocyanate [A] and 2,6-toluene diisocyanate [B] in the ratio 80:20 available from Covestro

### 1.2. Methods and Instrumentation

### 1.2.1. Solid content (By precipitation and separation method)

In a centrifuge tube 5g of polymer polyol were mixed with 25g of methanol. The resulting product was centrifuged at 12000 rpm for 30min at room temperature. The supernatant is removed and., the solid product that remains was dried under vacuum at 40°C for one hour. The solid product that remains was pulverized and dried again under vacuum at 40°C for two hours. The solid content (%) was determined by gravimetry.

### 1.2.2. Dynamic viscosity

Dynamic viscosity was determined following the standard EN ISO 3219, employing a Haake iQ viscometer using the spindle CC25DIN Viscosity determination according to this standard is performed at 25°C and 25 s-1.

### 1.2.3. Particle size

Particle size was determined by laser diffraction using a Mastersizer 3000 equipment dispersing the sample in ethanol and calculating the particle size distribution using Fraunhofer theory. d (0.1), d (0.5) and d (0.9) are the 10, 50 and 90% percentiles of the particle size distribution (volume). Span is the (d (0.9) - d (0.1)) / d (0.5) calculated value.

### 1.2.4. Reaction conversion

The reaction conversion for main monomers present expressed in % by weight was calculated by means of mass balance, considering the monomer in the feed (ACN and AMS) and the monomer in the flash and stripping vapor condensed product. The measurement is made as follows: $X % = \frac{\left(AMS+SM+ACN\right) in the flash and stripping}{\left(AMS+SM+ACN\right) in the feed} \times 100$

### 1.3. Preparation Process

### 1.3.1. Semibatch process

### Semibatch synthesis of polymer polyol of Ex. 1 of the present invention using polystyrene pyrolysis oil 1

Polymer polyol of Ex. 1 was prepared by a semibatch process using as a unique source of styrene monomer a pyrolysis oil 1 obtained from polystyrene. The process is disclosed herein below, wherein the amount of the reagents are expressed in % by weight in relation to the total weight of materials fed to the process.

In an autoclave reactor, part of the polyol (36% by weight) and the macromer A (3.8% by weight) was loaded. The reactor was closed, purged with nitrogen, and left to slight overpressure under nitrogen atmosphere (+0.8 barg). After that, the reactor was heated under stirring to 125°C.

Then, a solution composed of ACN monomer (15.5% by weight) and pyrolysis oil 1 (35.65% by weight; containing 82% of SM and 2.9% of AMS), free-radical initiator B (0.34% by weight), chain transfer agent (0.6% by weight, terpinolene) and polyol (7.91% by weight), was fed at room temperature and at a constant flow rate from the bottom of the reactor during a 120 min period of time. During the reaction, temperature was controlled to 125°C. Vapor outlet from reactor was closed, so reaction pressure increased as liquid level raised to a maximum of 3 barg. Polymerization was continued for 30 minutes after completion of semibatch feed time, at the same reaction temperature. Then, volatiles were removed under vacuum using nitrogen as stripping gas for 2 hours and 130°C and, condensed and collected in a vacuum trap using dry ice for mass balance calculation. Once the stripping of the reaction product was finished, it was cooled and discharged from the reactor for further analysis.

### Semibatch synthesis of polymer polyol of Ex. 2 of the present invention using fractionated polystyrene pyrolysis oil 2

Polymer polyol of Ex. 2 was prepared by a semibatch process using as a unique source of styrene monomer the fractionated pyrolysis oil 2 obtained from polystyrene. The process for the fractionation of the pyrolysis oil 1 as well as the process for the preparation of polymer polyol are disclosed herein below, wherein the amount of the reagents are expressed in % by weight in relation to the total weight of materials fed to the process.

### Fractionation of polystyrene pyrolysis Oil 1

Fractionation of the pyrolysis oil 1 in a column of 15 theoretical plates with a reflux ratio of 15:
- C7 light cut (up to 140°C, normal boiling point) at atmospheric pressure.
- C8 cut between 140-150°C normal boiling point (temperature equivalent to atmospheric pressure) at 100 mmHg.
- C9+ cut (Residue with normal boiling point > 150°C).

The fractionated pyrolysis oil 2 thus obtained comprises 92% by weight of styrene monomer and 3% of alpha methyl styrene as it is shown in the above-mentioned table in section 1.1. The resulting fractionated pyrolysis oil 2 was used as starting material in the synthesis of polymer polyol.

### Synthesis of Polymer polyol

In an autoclave reactor, part of the polyol (36.2% by weight) and the macromer A (3.75% by weight) was loaded. The reactor was closed, purged with nitrogen, and left to slight overpressure under nitrogen atmosphere (+0.8 barg). After that, the reactor was heated under stirring to 125°C.

Then, a solution composed of ACN monomer (14.0% by weight) and pyrolysis oil 1 (32.21% by weight; containing 92% of SM and 3% of AMS), free-radical initiator B (0.37% by weight), chain transfer agent (0.62% by weight, terpinolene) and the polyol A (12.86% by weight), was fed at room temperature and at a constant flow rate from the bottom of the reactor during a 120 min period of time. During the reaction, temperature was controlled to 125°C. Vapor outlet from reactor was closed, so reaction pressure increased as liquid level raised to a maximum of 3 barg. Polymerization was continued for 30 minutes after completion of semibatch feed time at the same reaction temperature. Then, volatiles were removed under vacuum using nitrogen as stripping gas for 2 hours and 130°C and, condensed and collected in a vacuum trap using dry ice for mass balance calculation. Once the stripping of the reaction product was finished, it was cooled and discharged from the reactor for further analysis.

### Semibatch synthesis of the comparative polymer polyol of Comparative Ex. 1 without the use of pyrolysis oil

Comparative Polymer polyol of comparative Ex. 1 was prepared by a semibatch process without the use of pyrolysis oil. In fact, the unique source of styrene monomer is a styrene monomer having a purity higher than 99,9%wt commercially available from Merck. The process is disclosed herein below, wherein the amount of the reagents are expressed in % by weight in relation to the total weight of materials fed to the process.

In an autoclave reactor, part of the polyol (43.3% by weight) and the macromer A (3.35% by weight) was loaded. The reactor was closed, purged with nitrogen, and left to slight overpressure under nitrogen atmosphere (+0.8 barg). After that, the reactor was heated under stirring to 125°C.

Then, a solution composed of styrene monomer (31% by weight) and ACN monomer (15.5% by weight), free-radical initiator B (0.34% by weight;), chain transfer agent (1.2% by weight, terpinolene) and the polyol (5.36% by weight), was fed at room temperature and at a constant flow rate from the bottom of the reactor during a 120 min period of time. During the reaction, temperature was controlled to 125°C. Vapor outlet from reactor was closed, so reaction pressure increased as liquid level raised to a maximum of 3 barg. Polymerization was continued for 30 minutes after completion of semibatch feed time at the same reaction temperature. Then, volatiles were removed under vacuum using nitrogen as stripping gas for 2 hours and 130°C and, condensed and collected in a vacuum trap using dry ice for mass balance calculation. Once the stripping of the reaction product was finished, it was cooled and discharged from the reactor for further analysis.

### 1.3.2. Continuous process

### Continuous synthesis of Comparative polymer polyol of Comparative Ex. 2 (without styrene pyrolysis oil)

The comparative polymer polyol Ex. 2 was prepared in two reactors connected in series, provided with stirrers and with temperature, flow and pressure control (backpressure control valve at the outlet of the second reactor). The second reactor is connected to the first one in series. A pre-mixed solution of the macromer B (2.82%wt), the styrene monomer (30.90%wt), the acrylonitrile (12.40%wt), the polyol (51.70%wt), the chain transfer agent (terpinolene, 0.52%wt) and the initiator B (0.43%wt) was pumped continuously into the first reactor in series. Reactor feed enters to the bottom of the reactor and exists from the top. Once stationary state was reached, reaction output polymer polyol product was collected from the second reactor in a stirred tank with a thermal jacket for heating and connection to a vacuum system, to perform flash and stripping of the final product of the reaction, in order to remove volatiles. Reaction was performed at 125°C, 3 barg and 30 minutes residence time per reactor.

### 1.4. Characterization of the polymer polyols

The solid content in percentage by weight of the polymer polyol of Examples 1 and 2 of the present invention and also of the comparative polymer polyol of Comparative Ex. 1 and 2 falling outside the scope of the present invention is disclosed in the following table herein below:

| **Parameter** | **Polymer polyol** | | | |
|---|---|---|---|---|
| | **Semibatch process** | | | **Continuous process** |
| | **Ex.1** | **Ex. 2** | **Comparative Ex.1** | **Comparative Ex.2** |
| Solid Content (%wt) | 44.1 | 42 | 43.5 | 40.5 |
| Viscosity (cp) | 3288 | 4002 | 3797 | 4000 |
| d (0.1) (micrometer) | 0.21 | 0.151 | 0.174 | 0.35 |
| d (0.5) (micrometer) | 0.365 | 0.27 | 0.302 | 0.848 |
| d (0.9) (micrometer) | 0.611 | 0.45 | 0.572 | 2.07 |
| span | 1.099 | 1.107 | 1.318 | 2.027 |
| Conversion (%) | 95.1 | 93 | 92.6 | 86.1 |

Regarding the polymer polyol, as it is shown in the values of the Table above, the polymer polyol of the present invention (Examples 1 and 2) which were prepared using polystyrene pyrolysis oil as source of styrene monomer has comparable properties/quality than those obtained using "pure" styrene monomer. Particularly, the particle size, viscosity and solid content were appropriate for having the quality and processability properties for being used in the preparation of compositions containing them such as polyurethane foams without compromising their final properties.

Particularly, it was surprisingly observed that the viscosity value of the polymer polyol obtained by the use of the polystyrene pyrolysis oil 1 is lower than those obtained with the comparative process. Therefore, the polymer polyol of the invention (cf. Ex.2) has better processability properties for the preparation of polyurethane foams.

Thus, considering all the experimental data provided in the present application, it is demonstrated that the use of pyrolysis oil (from polystyrene) does not affect negatively to the properties of the polymer polyol thus obtained (even observing enhanced properties), even when the pyrolysis oil is used without further fractioning/purification steps.

Besides, regarding the preparation process of the invention involving the use of pyrolysis oil, contrary to those expected/disclosed in the state of the art, no impact in both the quantitative conversion of the monomers and the quality of the polymer polyol was observed. In fact, a significantly reduction of the amount of (external) chain transfer agent added in the reaction mixture without the need of increasing the amount of the radical initiators is particularly observed without affecting the reaction conversion and the properties of the final polymer polyol and subsequent polyurethane foams. Furthermore, in comparison to the styrene monomer purification processes of the state of the art, the process of the present invention allows eliminating the volatile and styrene monomer co-boiler impurity compounds present in the polystyrene pyrolysis oil from the resulting polymer polyol crude mixture obtained when using polystyrene pyrolysis oil as styrene monomer source by a simple stripping, which allows reducing the tedious/complex purifying steps.

### 2. Polyurethane foams

### 2.1. Foam formulations

Polymer polyols of the present invention of Examples 1 and 2; and comparative polymer polyol of Comparative Example 2 were used as starting material for the preparation of polyurethane foams of Examples 1 and 2, and comparative Foam of Comparative Example 3.

The reagents used as starting materials for the preparation of the foam formulations and their amount expressed in parts per hundred parts of polyol (pphp) are disclosed herein below:

| **Ingredients** | **Foam No.** | | | |
|---|---|---|---|---|
| | **Foam Ex. 1** | **Foam Ex. 2** | **Comparative Foam Comparative Ex. 3** | |
| Polymer Polyol | Polymer polyol Example | Polymer polyol Ex. 1 | Polymer polyol Ex. 2 | comparative polymer polyol comparative Ex. 2 |
| | Amount (pphp) | 100 | 100 | 100 |
| Water (pphp) | | 1,25 | 1,25 | 1,25 |
| Niax^{®}DMEA (pphp) | | 0,25 | 0,25 | 0,25 |
| Niax^{®} A-107 (pphp) | | 0,2 | 0,2 | 0,2 |
| Kosmos^{®}29 (pphp) | | 0,13 | 0,13 | 0,13 |
| Tegostab^{®}B8002 (pphp) | | 1,3 | 1,3 | 1,3 |
| TDI T80/20 (pphp) | | 19,35 | 19,53 | 20,03 |
| Isocyanate (index) | | 115 | 115 | 115 |

### 2.2. Preparation process

The polyurethane foams were prepared following the process disclosed herein below:
The polymer polyol, water, amines catalyst (Niax^{®} DMEA and Niax^{®} Catalyst A-107), stannous octoate catalyst (Kosmos^{®}29) and surfactant silicone (Tegostab^{®} B8002) were added in the relation to 500 gr of Polyol in a 1 litter container. The contents were mixed at 2.000 rpm for 60 seconds with an agitator. The mixture thus obtained was then degassed for 10 seconds. While the mixer was still rotating, the amount needed of toluene diisocyanate was added to the container and the contents were mixed at 2.000 rpm for 10 seconds. The mixture was then poured into a 25x25 cm square box test, where the foam rise until the reaction was complete. The foam was then heated in an oven at 150 °C for 30 minutes. Subsequently, the foam was allowed to cure for 24 hours at room temperature before proceeding to its evaluation.

### 2.3. Characterization of foam obtained from polymer polyols

Foam properties and methods used for their determination are indicated herein below:

| **Foam No.** | **method** | **Foam Ex.1** | **Foam Ex. 2** | **Comparative Foam Comp Ex. 3** |
|---|---|---|---|---|
| Density (kg/m3) | ASTM D3574 | 58,8 | 59,7 | 57,4 |
| CLD 25% ( kPa ) | ISO 3386-1 | 11,95 | 10,52 | 10,4 |
| CLD 40% ( kPa ) | ISO 3386-1 | 14,68 | 12,90 | 12,72 |
| CLD 65% ( kPa ) | ISO 3386-1 | 34,73 | 29,63 | 29,8 |
| Resilience ( % ) | ASTM D3574 | 38 | 39 | 42 |
| Compression set 75% | ISO 1856 | 3,6 | 3,8 | 2,5 |
| Permeability ( mm/s) | ISO 9237 | 698 | 770 | 677 |

As it is known in the state of the art, the preparation of polyurethane, and particularly of polyurethane foams, is a highly complex chemical process, where several reactions occur simultaneously and with different catalytic processes. These reactions are very sensitive to different impurities (water, substances with active hydrogens, acids, bases, metals, among others), causing from the collapse of the foam to malformations of the cellular structure. Despite this, as it is shown in Table above, the foams obtained by the use of polymer polyol of the present invention (Examples 1 and 2) which has been prepared using polystyrene pyrolysis oil as source of styrene monomer has comparable values, and therefore properties, as those foams obtained using "pure" styrene monomer (Foam Comparative Ex. 3). Therefore, it is concluded that the use of polystyrene pyrolysis oil does surprisingly neither affect negatively to the properties of the polyurethane foams obtained from the polymer polyol of the present invention, even when the pyrolysis oil is directly used without further fractionating/purifying steps.

### Citation List

1. WO2014/035782
2. ASTM D3574
3. ISO 3386-1
4. ISO 1856
5. ISO 9237
6. John Scheirs, and Walter Kaminsky. "Feedstock Recycling and Pyrolysis of Waste Plastics: Converting Waste Plastics into Diesel and Other Fuels", Wiley Online Library, 2006, p635.
7. Jasmin Shah et al. "Conversion of waste polystyrene through catalytic degradation into valuable products", Korean J. Chem. Eng., 2014, vol. 31(8), pp.1389-1398.
8. US 4454255
9. Recycling of Polymers. Methods, Characterization and Applications. Ed. Raju Francis. 2017 Wiley-VCH. p. 81
10. EN ISO 3219.
11. EPA 3051a
12. ASTM D6052-97
13. US10301235
14. WO2021230312
15. WO2021053074
16. Ki-bum Park et al. "Two-stage pyrolysis of polystyrene: Pyrolysis oil as a source of fuel or benzene, toluene, ethylbenzene, and xylenes". Applied Energy 259, 2020, pp. 114240.
17. Ibrahim M. Maafa. "Pyrolysis of Polystyrene Waste: A review". Polymers, 2021, vol. 13, pp. 225.

## Claims

1. A polymer polyol comprising:
(a) a dispersed phase formed by polymeric particles (a1) which are the polymerization product of a polystyrene pyrolysis oil, and acrylonitrile; or alternatively, of a polystyrene pyrolysis oil, acrylonitrile, and styrene monomer;
and
(b) a continuous phase comprising:
(b1) a polyol; and
(b2) optionally, one or more of the compounds selected from the group consisting of macromer; dispersant; 2,4-diphenyl-1-butene and isomers thereof; 2,4,6-triphenyl-1-hexene and isomers thereof; 2-phenylnaphthalene; and poly-aromatic compounds;
wherein the polystyrene pyrolysis oil comprises from 50% to 98% by weight of styrene monomer,
wherein:
the polymeric particles (a1) comprise:
monomeric units of styrene, acrylonitrile, and alpha-methyl styrene; and
one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and
isomers thereof.

2. The polymer polyol according to claim 1, wherein the polyol is selected from the group consisting of polyether polyol, polyester polyol, polycarbonate polyol, polyether carbonate polyol and a mixture thereof.

3. The polymer polyol according to any of the claims 1 or 2, wherein the polyol is a polyether polyol.

4. The polymer polyol according to any of the claims 1-3, wherein the particle size of the polymeric particles is from 0.1 to 10µm measured by laser diffraction.

5. The polymer polyol according to any of the claims 1-4, which comprises from 10 to 65% by weight of polymeric particles in relation to the total weight of the polymer polyol.

6. The polymer polyol according to any of the claims 1-5, wherein the polymeric particles comprise:
from 20 to 80% by weight of styrene in relation to the total weight of the polymeric particles;
from 14 to 50 % by weight of acrylonitrile in relation to the total weight of the polymeric particles;
from 2 to 40 % by weight of alpha-methyl styrene in relation to the total weight of the polymeric particles; and
from 0.1 to 10% by weight of one or more monomeric units selected from the group consisting of propenyl benzene; divinylbenzene; phenylacetylene; methyl butenes; methyl pentenes; isobutene; pentenes; hexene; heptenes; octenes, cyclopentadiene;
allylbenzene; methyl-styrene other than alpha-methyl-styrene; ethyl-styrene; stilbene; 2,4-diphenyl-1-butene and isomers thereof; 1,4-diphenyl-1,3-butadiene and isomers thereof;
and 2,4,6-triphenyl-1-hexene and isomers thereof in relation to the total weight of the polymeric particles;
being the sum of the monomers up to 100% by weight of the polymeric particles.

7. The polymer polyol according to any of the claims 1-6, which comprises from 35 to 90% by weight of the polyol (b1) in relation to the total weight of the polymer polyol.

8. The polymer polyol according to any of the claims 1-7, wherein the polyol (b1) is a polyether polyol comprising propylene oxide and ethylene oxide units.

9. The polymer polyol according to any of the claims 1-8, wherein the polymer polyol has a dynamic viscosity from 500 cp to 30000 cp measured according to the standard EN ISO 3219.

10. A process for the preparation of a polymer polyol comprising:
(a) a dispersed phase formed by polymeric particles (a1); and
(b) a continuous phase comprising a polyol (b1);
wherein:
the polymeric particles (a1) comprise:
monomeric units of styrene, acrylonitrile, and alpha-methyl styrene; and
one or more monomeric units selected from the group consisting of propenyl benzene; divinylbenzene; phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene, and isomers thereof; and 2,4,6-triphenyl-1-hexene and isomers thereof;
and wherein the process comprises:
(i) providing polymeric particles dispersed in a continuous polyol phase by polymerization of a polystyrene pyrolysis oil, and acrylonitrile; or alternatively by polymerization of a polystyrene pyrolysis oil, acrylonitrile, and styrene monomer; and one or more monomeric units selected from the group consisting of propenyl benzene; divinylbenzene;
phenylacetylene; methyl butenes; methyl pentenes; isobutene; pentenes; hexene; heptenes; octenes, cyclopentadiene; allylbenzene; methyl-styrene other than alpha-methyl-styrene; ethyl-styrene; stilbene; 2,4-diphenyl-1-butene and isomers thereof; 1,4-diphenyl-1,3-butadiene and isomers thereof; and 2,4,6-triphenyl-1-hexene and isomers thereof;
in a continuous polyol phase in the presence of at least one radical initiator; wherein the polystyrene pyrolysis oil comprises from 50% to 98% by weight of styrene monomer.

11. The process according to claim 10, wherein the process further comprises an additional previous step (ii) before step (i) which comprises providing a polystyrene pyrolysis oil comprising from 50% to 98% by weight of styrene monomer by submitting one or more polystyrene compounds and/or one or more polystyrene containing compounds to a pyrolysis reaction.

12. The process according to any of the claims 10 or 11, wherein the styrene pyrolysis oil comprises:
from 50 to 98% by weight of styrene monomer;
from 0.1 to 10% by weight of alpha-methyl styrene;
from 0.0005 to 0.1% by weight of phenylacetylene;
from 0.2 to 15% by weight of toluene;
from 0 to 5% by weight of benzene;
from 0.5 to 12% by weight of C₈ aromatic compounds;
from 0.1 to 3% by weight of C₉ aromatic compounds;
from 0.01 to 1% by weight of C₁₀ aromatic compounds; particularly selected from the group consisting of divinylbenzene, ethyl-styrene and a mixture thereof;
from 0 to 1% by weight of a (C₃-C₈)fraction comprising one or more compounds selected form the group consisting of (C₃-C₈)alkanes, (C₃-C₈)alkenes, (C₅-C₈)cycloalkanes and (C₅-C₈)cycloalkenes;
from 0 to 15% by weight of a heavy oil fraction; and
from 0.5 to 5000ppm of heteroatom containing compounds;
particularly, wherein the styrene pyrolysis oil comprises:
from 50 to 98% by weight of styrene monomer;
from 0.1 to 10% by weight of alpha-methyl styrene;
from 0.0005 to 0.1% by weight of phenylacetylene;
from 0.2 to 15% by weight of toluene;
from 0 to 5% by weight of benzene;
from 0.5 to 12% by weight of C₈ aromatic compounds;
from 0.1 to 3% by weight of C₉ aromatic compounds;
from 0.01 to 1% by weight of C₁₀ aromatic compounds; particularly selected from the group consisting of divinylbenzene, ethyl-styrene and a mixture thereof;
from 0 to 1% by weight of a (C₃-C₈)fraction comprising one or more compounds selected form the group consisting of (C₃-C₈)alkanes, (C₃-C₈)alkenes, (C₅-C₈)cycloalkanes and (C₅-C₈)cycloalkenes;
from 0 to 15% by weight of a heavy oil fraction; and
from 0.5 to 5000ppm of heteroatom containing compounds; and
the polymer polyol is as defined in any of the claims 1-10.

13. A process for the preparation of a polyurethane, comprising the reaction of at least one polymer polyol as defined in any of the claims 1-9 with at least one di- or poly-isocyanate; and at least a blowing agent.

14. A composition comprising the polymer polyol as defined in any of the claims 1-9 and one or more excipients or carriers; or alternatively,
a composition made at least from the polymer polyol as defined in any of the claims 1-9;
particularly, the composition made at least from the polymer polyol as defined in any of the claims 1-9 is a polyurethane; and more particularly, a polyurethane foam.

## Patentansprüche

1. Ein Polymerpolyol, umfassend:
(a) eine dispergierte Phase, gebildet aus Polymerpartikeln (a1), welche das Polymerisationsprodukt aus einem Polystyrolpyrolyseöl und Acrylnitril; oder alternativ aus einem Polystyrolpyrolyseöl, Acrylnitril und Styrolmonomer sind;
und
(b) eine kontinuierliche Phase, umfassend:
(b1) ein Polyol; und
(b2) wahlweise eine oder mehrere der Verbindungen, die ausgewählt sind aus der Gruppe bestehend aus Makromer; Dispergiermittel; 2,4-Diphenyl-1-buten und Isomeren davon; 2,4,6-Triphenyl-1-hexen und Isomeren davon; 2-Phenylnaphthalin; und Polyaromaten;
wobei das Polystyrolpyrolyseöl von 50 bis 98 Gew.- % Styrolmonomer umfasst,
wobei:
die Polymerpartikel (a1) Folgendes umfassen:
monomere Einheiten von Styrol, Acrylnitril und alpha-Methylstyrol; und
eine oder mehrere monomere Einheiten, die ausgewählt sind aus der Gruppe, die aus Propenylbenzol; Divinylbenzol; Phenylacetylen; Methylbutenen; Methylpentenen;
Isobuten; Pentenen; Hexen; Heptenen; Octenen, Cyclopentadien; Allylbenzol; von alpha-Methylstyrol verschiedenem Methylstyrol; Ethylstyrol; Stilben; 2,4-Diphenyl-1-buten und
Isomeren davon; 1,4-Diphenyl-1,3-butadien und Isomeren davon; und 2,4,6-Triphenyl-1-hexen und Isomeren davon besteht.

2. Das Polymerpolyol nach Anspruch 1, wobei das Polyol ausgewählt ist aus der Gruppe bestehend aus Polyetherpolyol, Polyesterpolyol, Polycarbonatpolyol, Polyethercarbonatpolyol und einer Mischung davon.

3. Das Polymerpolyol nach einem der Ansprüche 1 oder 2, wobei das Polyol ein Polyetherpolyol ist.

4. Das Polymerpolyol nach einem der Ansprüche 1 bis 3, wobei die Partikelgröße der Polymerpartikel, gemessen durch Laserbeugung, von 0,1 bis 10 µm beträgt.

5. Das Polymerpolyol nach einem der Ansprüche 1 bis 4, welches von 10 bis 65 Gew.- % Polymerteilchen in Bezug auf das Gesamtgewicht des Polymerpolyols umfasst.

6. Das Polymerpolyol nach einem der Ansprüche 1 bis 5, wobei die Polymerteilchen Folgendes umfassen:
von 20 bis 80 Gew.- % Styrol, bezogen auf das Gesamtgewicht der Polymerpartikel;
von 14 bis 50 Gew.- % Acrylnitril, bezogen auf das Gesamtgewicht der Polymerpartikel;
von 2 bis 40 Gew.- % alpha-Methylstyrol, bezogen auf das Gesamtgewicht der polymeren Partikeln; und
von 0,1 bis 10 Gew.- % einer oder mehrerer monomerer Einheiten, die ausgewählt sind aus der Gruppe bestehend aus Propenylbenzol; Divinylbenzol; Phenylacetylen;
Methylbutenen; Methylpentenen; Isobuten; Pentenen; Hexen; Heptenen; Octenen, Cyclopentadien; Allylbenzol; von alpha-Methylstyrol verschiedenem Methylstyrol;
Ethylstyrol; Stilben; 2,4-Diphenyl-1-buten und Isomeren davon; 1,4-Diphenyl-1,3-butadien und Isomeren davon; und 2,4,6-Triphenyl-1-hexen und Isomeren davon, bezogen auf das Gesamtgewicht der Polymerpartikel;
wobei die Summe der Monomere bis zu 100 Gew.- % der Polymerpartikel beträgt.

7. Das Polymerpolyol nach einem der Ansprüche 1 bis 6, welches von 35 bis 90 Gew.- % des Polyols (b1) in Bezug auf das Gesamtgewicht des Polymerpolyols umfasst.

8. Das Polymerpolyol nach einem der Ansprüche 1 bis 7, wobei das Polyol (b1) ein Polyetherpolyol ist, das Propylenoxid- und Ethylenoxideinheiten umfasst.

9. Das Polymerpolyol nach einem der Ansprüche 1 bis 8, wobei das Polymerpolyol eine dynamische Viskosität von 500 cp bis 30000 cp, gemessen nach der Norm EN ISO 3219, hat.

10. Ein Verfahren zur Herstellung eines Polymerpolyols, umfassend:
(a) eine dispergierte Phase, die aus Polymerpartikeln (a1) besteht; und
(b) eine kontinuierliche Phase, die ein Polyol (b1) umfasst;
wobei:
die Polymerpartikel (a1) Folgendes umfassen:
monomere Einheiten von Styrol, Acrylnitril und alpha-Methylstyrol; und
eine oder mehrere monomere Einheiten, die aus der Gruppe ausgewählt sind, die aus Propenylbenzol; Divinylbenzol; Phenylacetylen; Methylbutenen; Methylpentenen; Isobuten; Pentenen; Hexen; Heptenen; Octenen, Cyclopentadien; Allylbenzol; von alpha-Methylstyrol verschiedenem Methylstyrol; Ethylstyrol; Stilben; 2,4-Diphenyl-1-buten und Isomeren davon; 1,4-Diphenyl-1,3-butadien und Isomeren davon; und 2,4,6-Triphenyl-1-hexen und Isomeren davon besteht;
und wobei das Verfahren Folgendes umfasst:
(i) Bereitstellen von Polymerpartikeln, die in einer kontinuierlichen Polyolphase dispergiert sind, durch Polymerisation eines Polystyrolpyrolyseöls und Acrylnitrils; oder alternativ durch Polymerisation von einem Polystyrolpyrolyseöl, Acrylnitril und Styrolmonomer; und einer oder mehrerer monomerer Einheiten, die ausgewählt sind aus der Gruppe bestehend aus Propenylbenzol; Divinylbenzol; Phenylacetylen; Methylbutenen;
Methylpentenen; Isobuten; Pentenen; Hexen; Heptenen; Octenen, Cyclopentadien; Allylbenzol; von alpha-Methylstyrol verschiedenem Methylstyrol; Ethylstyrol; Stilben; 2,4-Diphenyl-1-buten und Isomeren davon; 1,4-Diphenyl-1,3-butadien und Isomeren davon; und 2,4,6-Triphenyl-1-hexen und Isomeren davon;
in einer kontinuierlichen Polyolphase in Gegenwart von mindestens einem Radikalinitiator; wobei das Polystyrolpyrolyseöl von 50 bis 98 Gew.- % Styrolmonomer umfasst.

11. Das Verfahren nach Anspruch 10, wobei das Verfahren ferner einen zusätzlichen vorhergehenden Schritt (ii) vor Schritt (i) umfasst, welcher das Bereitstellen eines Polystyrolpyrolyseöls umfasst, das von 50 bis 98 Gew.- % Styrolmonomer umfasst, indem eine oder mehrere Polystyrolverbindungen und/oder eine oder mehrere Polystyrol enthaltende Verbindungen einer Pyrolysereaktion unterzogen werden.

12. Das Verfahren nach einem der Ansprüche 10 oder 11, wobei das Styrolpyrolyseöl Folgendes umfasst:
von 50 bis 98 Gew.- % Styrolmonomer;
von 0,1 bis 10 Gew.- % alpha-Methylstyrol;
von 0,0005 bis 0,1 Gew.- % Phenylacetylen;
von 0,2 bis 15 Gew.- % Toluol;
von 0 bis 5 Gew.- % Benzol;
von 0,5 bis 12 Gew.- % C₈-Aromaten;
von 0,1 bis 3 Gew.- % C₉-Aromaten;
von 0,01 bis 1 Gew.- % C₁₀-Aromaten; insbesondere ausgewählt aus der Gruppe bestehend aus Divinylbenzol, Ethylstyrol und einer Mischung davon;
von 0 bis 1 Gew.- % einer (C₃-C₈)-Fraktion, die eine oder mehrere Verbindungen umfasst,
die aus der Gruppe ausgewählt sind, die aus (C₃-C₈)-Alkanen, (C₃-C₈)-Alkenen, (C₅-C₈)-Cycloalkanen und (C₅-C₈)-Cycloalkenen besteht;
von 0 bis 15 Gew.- % einer Schwerölfraktion; und
von 0,5 bis 5000 ppm heteroatomhaltige Verbindungen;
insbesondere, wobei das Styrolpyrolyseöl Folgendes umfasst:
von 50 bis 98 Gew.- % Styrolmonomer;
von 0,1 bis 10 Gew.- % alpha-Methylstyrol;
von 0,0005 bis 0,1 Gew.- % Phenylacetylen;
von 0,2 bis 15 Gew.- % Toluol;
von 0 bis 5 Gew.- % Benzol;
von 0,5 bis 12 Gew.- % C₈-Aromaten;
von 0,1 bis 3 Gew.- % C₉-Aromaten;
von 0,01 bis 1 Gew.- % C₁₀-Aromaten; insbesondere ausgewählt aus der Gruppe bestehend aus Divinylbenzol, Ethylstyrol und einer Mischung davon;
von 0 bis 1 Gew.- % einer (C₃-C₈)-Fraktion, die eine oder mehrere Verbindungen umfasst,
die aus der Gruppe ausgewählt sind, die aus (C₃-C₈)-Alkanen, (C₃-C₈)-Alkenen, (C₅-C₈)-Cycloalkanen und (C₅-C₈)-Cycloalkenen besteht;
von 0 bis 15 Gew.- % einer Schwerölfraktion; und
von 0,5 bis 5000 ppm heteroatomhaltige Verbindungen; und
das Polymerpolyol wie in einem der Ansprüche 1 bis 10 definiert ist.

13. Ein Verfahren zur Herstellung eines Polyurethans, umfassend die Umsetzung von mindestens einem Polymerpolyol, wie in einem der Ansprüche 1 bis 9 definiert, mit mindestens einem Di- oder Polyisocyanat; und mindestens einem Treibmittel.

14. Eine Zusammensetzung umfassend das Polymerpolyol wie in einem der Ansprüche 1 bis 9 definiert und einen oder mehrere Hilfsstoffe oder Träger; oder alternativ eine Zusammensetzung, die mindestens aus dem Polymerpolyol wie in einem der Ansprüche 1 bis 9 definiert hergestellt ist;
insbesondere ist die Zusammensetzung, die mindestens aus dem Polymerpolyol wie in einem der Ansprüche 1 bis 9 definiert hergestellt ist, ein Polyurethan; und, ganz besonders, ein Polyurethanschaum.

## Revendications

1. Un polyol polymère comprenant :
(a) une phase dispersée formée par des particules polymères (a1) qui sont le produit de polymérisation d'une huile de pyrolyse de polystyrène et d'acrylonitrile ; ou, en variante, d'une huile de pyrolyse de polystyrène, d'acrylonitrile et de monomère de styrène ;
et
(b) une phase continue comprenant :
(b1) un polyol ; et
(b2) éventuellement, un ou plusieurs des composés choisis dans le groupe constitué par un macromère ; un dispersant ; le 2,4-diphényl-1-butène et les isomères de celui-ci ; le 2,4,6-triphényl-1-hexène et les isomères de celui-ci ; le 2-phénylnaphtalène ; et les composés poly-aromatiques ; dans lequel l'huile de pyrolyse de polystyrène comprend de 50 % à 98 % en poids de monomère de styrène,
dans lequel :
les particules polymères (a1) comprennent :
des unités monomères de styrène, d'acrylonitrile et d'alpha-méthylstyrène ; et
une ou plusieurs unités monomères choisies dans le groupe constitué par le propénylbenzène ; le divinylbenzène ; le phénylacétylène ; les méthylbutènes ; les méthylpentènes ; l'isobutène ; les pentènes ; l'hexène ; les heptènes ; les octènes, le cyclopentadiène ; l'allylbenzène ; le méthylstyrène autre que l'alpha-méthylstyrène ;
l'éthylstyrène ; le stilbène ; le 2,4-diphényl-1-butène et les isomères de celui-ci ; le 1,4-diphényl-1,3-butadiène et les isomères de celui-ci ; et le 2,4,6-triphényl-1-hexène et les isomères de celui-ci.

2. Le polyol polymère selon la revendication 1, dans lequel le polyol est choisi dans le groupe constitué par le polyéther polyol, le polyester polyol, le polycarbonate polyol, le polyéther carbonate polyol et un mélange de ceux-ci.

3. Le polyol polymère selon l'une quelconque des revendications 1 ou 2, dans lequel le polyol est un polyéther polyol.

4. Le polyol polymère selon l'une quelconque des revendications 1 à 3, dans lequel la taille de particule des particules polymères est de 0,1 à 10 µm mesurée par diffraction laser.

5. Le polyol polymère selon l'une quelconque des revendications 1 à 4, qui comprend de 10 à 65 % en poids de particules polymères par rapport au poids total du polyol polymère.

6. Le polyol polymère selon l'une quelconque des revendications 1 à 5, dans lequel les particules polymères comprennent :
de 20 à 80 % en poids de styrène par rapport au poids total des particules polymères ;
de 14 à 50 % en poids d'acrylonitrile par rapport au poids total des particules polymères ;
de 2 à 40 % en poids d'alpha-méthylstyrène par rapport au poids total des particules polymères ; et
de 0,1 à 10 % en poids d'une ou plusieurs unités monomères choisies dans le groupe constitué par le propénylbenzène ; le divinylbenzène ; le phénylacétylène ; les méthylbutènes ; les méthylpentènes ; l'isobutène ; les pentènes ; l'hexène ; les heptènes ;
les octènes, le cyclopentadiène ; l'allylbenzène ; le méthylstyrène autre que l'alpha-méthylstyrène ; l'éthylstyrène ; le stilbène ; le 2,4-diphényl-1-butène et les isomères de celui-ci ; le 1,4-diphényl-1,3-butadiène et les isomères de celui-ci ; et le 2,4,6-triphényl-1-hexène et les isomères de celui-ci par rapport au poids total des particules polymères ;
étant la somme des monomères jusqu'à 100 % en poids des particules polymères.

7. Le polyol polymère selon l'une quelconque des revendications 1 à 6, qui comprend de 35 à 90 % en poids du polyol (b1) par rapport au poids total du polyol polymère.

8. Le polyol polymère selon l'une quelconque des revendications 1 à 7, dans lequel le polyol (b1) est un polyol de polyéther comprenant des unités d'oxyde de propylène et d'oxyde d'éthylène.

9. Le polyol polymère selon l'une quelconque des revendications 1 à 8, dans lequel le polyol polymère a une viscosité dynamique de 500 cp à 30 000 cp mesurée selon la norme EN ISO 3219.

10. Un procédé de préparation d'un polyol polymère comprenant :
(a) une phase dispersée formée de particules polymères (a1) ; et
(b) une phase continue comprenant un polyol (b1) ;
dans lequel :
les particules polymères (a1) comprennent :
des unités monomères de styrène, d'acrylonitrile et d'alpha-méthylstyrène ; et
une ou plusieurs unités monomères choisies dans le groupe constitué par le propénylbenzène ; le divinylbenzène ; le phénylacétylène ; les méthylbutènes ; les méthylpentènes ; l'isobutène ; les pentènes ; l'hexène ; les heptènes ; les octènes, le cyclopentadiène ; l'allylbenzène ; le méthylstyrène autre que l'alpha-méthylstyrène ; l'éthylstyrène ; le stilbène ; le 2,4-diphényl-1-butène et les isomères de celui-ci ; le 1,4-diphényl-1,3-butadiène et les isomères de celui-ci ; et le 2,4,6-triphényl-1-hexène et les isomères de celui-ci ;
et dans lequel le procédé comprend :
(i) fournir des particules polymères dispersées dans une phase de polyol continue par polymérisation d'une huile de pyrolyse de polystyrène et d'acrylonitrile ; ou, en variante, par polymérisation d'une huile de pyrolyse de polystyrène, d'acrylonitrile et de monomère de styrène ; et une ou plusieurs unités monomères choisies dans le groupe constitué par le propénylbenzène ; le divinylbenzène ; le phénylacétylène ; les méthylbutènes ; les méthylpentènes ; l'isobutène ; les pentènes ; l'hexène ; les heptènes ; les octènes, le cyclopentadiène ; l'allylbenzène ; le méthylstyrène autre que l'alpha-méthylstyrène ;
l'éthylstyrène ; le stilbène ; le 2,4-diphényl-1-butène et les isomères de celui-ci ; le 1,4-diphényl-1,3-butadiène et les isomères de celui-ci ; et le 2,4,6-triphényl-1-hexène et les isomères de celui-ci ;
dans une phase de polyol continue en présence d'au moins un initiateur radicalaire ; dans lequel l'huile de pyrolyse de polystyrène comprend de 50 % à 98 % en poids de monomère de styrène.

11. Le procédé selon la revendication 10, dans lequel le procédé comprend en outre une étape précédente supplémentaire (ii) avant l'étape (i) qui comprend la fourniture d'une huile de pyrolyse de polystyrène comprenant de 50 % à 98 % en poids de monomère de styrène en soumettant un ou plusieurs composés de polystyrène et/ou un ou plusieurs composés contenant du polystyrène à une réaction de pyrolyse.

12. Le procédé selon l'une quelconque des revendications 10 ou 11, dans lequel l'huile de pyrolyse de styrène comprend :
de 50 à 98 % en poids de monomère de styrène ;
de 0,1 à 10 % en poids d'alpha-méthylstyrène ;
de 0,0005 à 0,1 % en poids de phénylacétylène ;
de 0,2 à 15 % en poids de toluène ;
de 0 à 5 % en poids de benzène ;
de 0,5 à 12 % en poids de composés aromatiques en C₈ ;
de 0,1 à 3 % en poids de composés aromatiques en C₉ ;
de 0,01 à 1 % en poids de composés aromatiques en C₁₀ ; en particulier choisis dans le groupe constitué par le divinylbenzène, l'éthylstyrène et un mélange de ceux-ci ;
de 0 à 1 % en poids d'une fraction (C₃-C₈) comprenant un ou plusieurs composés choisis dans le groupe constitué par les alcanes en (C₃-C₈), les alcènes en (C₃-C₈), les cycloalcanes en (C₅-C₈) et les cycloalcènes en (C₅-C₈);
de 0 à 15 % en poids d'une fraction d'huile lourde ; et
de 0,5 à 5000 ppm de composés contenant des hétéroatomes ;
en particulier, dans lequel l'huile de pyrolyse de styrène comprend :
de 50 à 98 % en poids de monomère de styrène ;
de 0,1 à 10 % en poids d'alpha-méthylstyrène ;
de 0,0005 à 0,1 % en poids de phénylacétylène ;
de 0,2 à 15 % en poids de toluène ;
de 0 à 5 % en poids de benzène ;
de 0,5 à 12 % en poids de composés aromatiques en C₈ ;
de 0,1 à 3 % en poids de composés aromatiques en C₉ ;
de 0,01 à 1 % en poids de composés aromatiques en C₁₀ ; en particulier choisis dans le groupe constitué par le divinylbenzène, l'éthylstyrène et un mélange de ceux-ci ;
de 0 à 1 % en poids d'une fraction (C₃-C₈) comprenant un ou plusieurs composés choisis dans le groupe constitué par les alcanes en (C₃-C₈), les alcènes en (C₃-C₈), les cycloalcanes en (C₅-C₈) et les cycloalcènes en (C₅-C₈);
de 0 à 15 % en poids d'une fraction d'huile lourde ; et
de 0,5 à 5000 ppm de composés contenant des hétéroatomes ; et
le polyol polymère est tel que défini dans l'une quelconque des revendications 1 à 10.

13. Un procédé de préparation d'un polyuréthane, comprenant la réaction d'au moins un polyol polymère tel que défini dans l'une quelconque des revendications 1 à 9 avec au moins un di- ou poly-isocyanate ; et au moins un agent de gonflement.

14. Une composition comprenant le polyol polymère tel que défini dans l'une quelconque des revendications 1 à 9 et un ou plusieurs excipients ou véhicules ; ou, en variante, une composition fabriquée au moins à partir du polyol polymère tel que défini dans l'une quelconque des revendications 1 à 9 ;
en particulier, la composition fabriquée au moins à partir du polyol polymère tel que défini dans l'une quelconque des revendications 1 à 9 est un polyuréthane ; et plus particulièrement, une mousse de polyuréthane.
